# EUROPÄISCHE PATENTANMELDUNG

(11) **EP 4 389 161 A1**
(43) Veröffentlichungstag der Anmeldung: **26.06.2024**
(21) Anmeldenummer: 23166335.2
(22) Anmeldetag: 03.04.2023
(51) Int. Cl.: A61L 9/20

(54) **VORRICHTUNG ZUR DESINFEKTION VON LUFT**

(30) Priorität: 19.12.2022 DE 102022133831
(71) Anmelder: Purventus GmbH, 99098 Erfurt (DE)
(72) Erfinder: Krenz, Karlheinz, 99102 Klettbach (DE)
(74) Vertreter: Hertin und Partner Rechts- und Patentanwälte PartG mbB

(57) **Zusammenfassung**

Die Erfindung betrifft eine Vorrichtung zur Desinfektion von Luft. Die Vorrichtung umfasst eine Lufteinlasseinheit, eine Luftfördereinheit, eine Desinfektionseinheit und eine Luftauslasseinheit. Die Lufteinlasseinheit und die Luftfördereinheit ist für das Ansaugen von Ausatemluft von mindestens zwei Personen konfiguriert, die Luftfördereinheit ist für die Förderung der Ausatemluft von der Lufteinlasseinheit über die Desinfektionseinheit zu der Luftauslasseinheit konfiguriert und die Luftauslasseinheit ist für die Bildung einer Luftbarriere, vorzugsweise zwischen den mindestens zwei Personen, konfiguriert. Die Lufteinlasseinheit liegt in Umfangsrichtung um die Luftauslasseinheit angeordnet vor. Vorzugsweise so, dass mindestens ein Bereich der Lufteinlasseinheit zwischen jeder der mindestens zwei Personen und der Luftauslasseinheit positionierbar ist. Die Luftauslasseinheit ist mindestens teilweise von der Lufteinlasseinheit umgeben. Die Erfindung betrifft zudem ein System umfassend eine erfindungsgemäße Vorrichtung und ein Möbelstück. Weiterhin betrifft die Erfindung eine Verwendung der erfindungsgemäßen Vorrichtung zu einer Reduzierung eines Ansteckungsrisikos durch luftübertragene Viruserkrankungen zwischen mindestens zwei Personen, die sich sitzend oder stehend an einem Möbelstück befinden. Die Erfindung betrifft auch ein Verfahren zur Desinfektion von Luft unter Verwendung der erfindungsgemäßen Vorrichtung.

## Beschreibung

Die Erfindung betrifft eine Vorrichtung zur Desinfektion von Luft. Die Vorrichtung umfasst eine Lufteinlasseinheit, eine Luftfördereinheit, eine Desinfektionseinheit und eine Luftauslasseinheit. Die Lufteinlasseinheit und die Luftfördereinheit sind für das Ansaugen von Ausatemluft von mindestens zwei Personen konfiguriert, die Luftfördereinheit ist für die Förderung der Ausatemluft von der Lufteinlasseinheit über die Desinfektionseinheit zu der Luftauslasseinheit konfiguriert und die Luftauslasseinheit ist für die Bildung einer Luftbarriere, vorzugsweise zwischen den mindestens zwei Personen, konfiguriert. Die Lufteinlasseinheit liegt in Umfangsrichtung um die Luftauslasseinheit angeordnet vor. Vorzugsweise so, dass mindestens ein Bereich der Lufteinlasseinheit zwischen jeder der mindestens zwei Personen und der Luftauslasseinheit positionierbar ist. Die Luftauslasseinheit ist mindestens teilweise von der Lufteinlasseinheit umgeben.

Die Erfindung betrifft zudem ein System umfassend eine erfindungsgemäße Vorrichtung und ein Möbelstück. Weiterhin betrifft die Erfindung eine Verwendung der erfindungsgemäßen Vorrichtung zu einer Reduzierung eines Ansteckungsrisikos durch luftübertragene Viruserkrankungen zwischen mindestens zwei Personen, die sich sitzend oder stehend an einem Möbelstück befinden. Die Erfindung betrifft auch ein Verfahren zur Desinfektion von Luft unter Verwendung der erfindungsgemäßen Vorrichtung.

### Hintergrund und Stand der Technik

Die Erfindung betrifft das Gebiet der Luftreinigung.

Mit der ausgeatmeten Luft verbreitet jeder Mensch Aerosolpartikel in seiner unmittelbaren Umgebung. Beim Sprechen, Singen und insbesondere beim Husten, Niesen oder unter körperlicher Anstrengung werden vermehrt Flüssigkeitströpfchen ausgestoßen. Je nach Größe und Eigenschaften wird in größere Flüssigkeitströpfchen und kleinere Aerosole unterschieden. Während die größeren Tröpfchen schnell zu Boden sinken, können Aerosole auch über längere Zeit in der Luft schweben und sich in geschlossenen Räumen verteilen. Aerosole können aufgrund ihrer geringen Größe je nach Umgebungsbedingungen bis zu mehreren Stunden luftgetragen in den Innenräumen verbleiben.

Befinden sich Krankheitserreger, wie beispielsweise SARS-CoV-2-Viren, in den Atemwegen, so können die ausgestoßenen Flüssigkeitströpfchen auch diese Krankheitserreger enthalten. Eine Ansteckung kann dann erfolgen, wenn diese virushaltige Flüssigkeitströpfchen oder Aerosole an die Schleimhäute der Nase, des Mundes oder der Augen einer anderen Person gelangen. Folglich besteht ein erhöhtes Ansteckungsrisiko beim Zusammenkommen von Menschen in Innenräumen aufgrund der Übertragungsmöglichkeiten von Viren durch mit Viren belastete Aerosolteilchen. Zu solchen Innenräumen zählen beispielsweise Klassenräume in Schulen, Seminar- und Schulungsräumen, Hörsäle, Arztpraxen, Besprechungszimmer, Restaurants oder Fitnessstudios.

Aus der Praxis sind Geräte zur Desinfektion von Raumluft in unterschiedlichen Ausführungsformen und Funktionsweisen bekannt. So kommen Desinfektionsgeräte mit unterschiedlicher Funktionsweise wie beispielsweise mit Ionisator oder Plasma-Luftreiniger, mit UV-Strahlung oder Geräte mit Filtern wie HEPA- oder Aktivkohle zum Einsatz. So ist es bekannt, dass derartige Desinfektionsgeräte mit UV-C-Strahlern versehen werden können, um Keime (hier auch synonym als Krankheitserreger bezeichnet) wie etwa Bakterien, Sporen, Parasiten, Viren oder Viroide, Pilze oder Algen etc. aus der Raumluft zu inaktivieren bzw. abzutöten. Dabei wird die Raumluft durch ein solches Desinfektionsgerät geführt, dort der UV-C-Strahlung ausgesetzt und anschließend wieder dem entsprechenden Raum zugeführt. Der Wellenlängenbereich der dabei eingesetzte UV-C-Strahlung reicht typischerweise von 100 nm bis 280 nm. So können zum Beispiel Niederdruck-Quecksilberdampflampen eingesetzt werden, die Strahlung bzw. Licht einer solchen Wellenlänge emittieren.

Bei dem Einsatz in Innenräumen müssen Desinfektionsgeräte von ihren Leistungsmerkmalen wie zum Beispiel Luftdurchsatz und Reinigungsgrad der von dem Gerät abgegebenen desinfizierten Luft dem Raumvolumen angepasst sein. Um eine ausreichend desinfizierende Reinigungswirkung zur Minimierung des Ansteckungsrisikos im Innenraum sicherzustellen, muss die gesamte Innenraumluft mehrfach pro Stunde durch das Desinfektionsgerät geführt werden. Dabei resultiert gerade bei großvolumigen Innenräumen das Problem, dass entweder ein sehr leistungsstarkes und damit unhandlich großes Desinfektionsgerät oder aber eine Vielzahl von Desinfektionsgeräten eingesetzt werden müssen. Dies ist dabei mit einem hohen Energieverbrauch und einer erheblichen Geräuschemission verbunden. Für eine angenehme Arbeitsatmosphäre, so beispielsweise in Arbeits-, Besprechungs- Empfangs oder Untersuchungsräumen, gilt dabei eine möglichst niedrige Geräuschemission als erstrebenswert. Leistungsstarke Desinfektionsgeräte für große Raumvolumen und insbesondere druckmindernde Abreinigungsstufen durch integrierte Filterelemente, durch die die Luft geführt werden muss, zeichnen sich oftmals durch eine ausgeprägte Geräuschentwicklung aus.

Bekannt sind auch Desinfektionsgeräte, die nicht einen kompletten Raum desinfizieren, sondern eine keim-inaktivierte Raumzone erzielen (lokal-wirkend). Ziel ist dabei den Luftbereich von Personen zum jeweiligen Ein- und/oder Ausatmen so gezielt aufzubereiten, dass ein entsprechendes Ansteckungsrisiko mit Krankheitserregern minimiert ist. Da diese Geräte einen entsprechend deutlich kleineres Raumvolumen aufbereiten müssen, können diese energiesparsamer, effizienter und geräuschärmer sein.

Bekannt sind dabei derartige lokal-wirkende Desinfektionsgeräte, die das primäre Ziel haben, dass eine Art gereinigter Luftraum um den Kopf/Person mit desinfizierter Luft geschaffen wird. So soll die Person möglichst nur frisch desinfizierte Luft einatmen.

So beschreibt beispielsweise DE 10 2020 211 777 A1 ein Tischgerät zur Erzeugung einer im Wesentlichen keim-inaktivierten Raumzone. Die Ansaugleitung soll dabei Luft außerhalb dieser geschaffenen Raumzone aufnehmen. Die angesaugte Luft wird in den Innenraum des Gerätes geleitet, um Keime in der aufgenommenen Luft durch eine Strahlungsquelle im UV-C-Spektralbereich im Innenraum zu inaktivieren bzw. abzutöten. Die Luftauslassvorrichtung ist dabei ausgelegt, eine im Wesentlichen laminare bzw. verwirbelungsarme Strömung in der ausströmenden Luft auszubilden. Diese Strömung erzeugt die im Wesentlichen keim-inaktivierte (d.h., erheblich keim-reduzierte) Raumzone innerhalb des umgebenden Raums. Dadurch erfolgt eine stabile Abgrenzung gegenüber der die keim-inaktivierte Raumzone umgebenden und potentiell keimbelasteten Luft. Die keim-inaktivierte Raumzone ist folglich gegenüber der Umgebung abgeschlossen.

DE 197 42 358 A1 beschreibt ein transportables Kleinstluftreinigungsgerät, wobei das Kleinstluftreinigungsgerät eine als Strömungsleitkörper ausgebildete Ausströmvorrichtung aufweist, mittels welcher der Luftstrom als gerichteter Strahl im Wesentlichen unvermischt mit ungereinigter Luft auf einen Benutzer zur Schaffung einer räumlich begrenzten Atmosphäre für den Benutzer leitbar ist. Der gerichtete Reinluftstrom führt dabei dazu, dass eine Person, die sich im Strömungsbereich befindet, von der Strömung im Wesentlichen geschlossen umströmt wird. Aufgrund dieses Umströmungsverhaltens wird in vorteilhafter Weise um die Person herum eine Art Glocke schadstoffverminderter Reinluft aufgebaut und aufrechterhalten. Die Reinigung und Desinfektion erfolgt durch jeweils sukzessive im Luftstrom angeordnete Mikrofeinstfilter, UV-Lampen und Kohleaktivfilterelemente.

DE 20 2020 103 935 beschreibt einen Atemluftspender mit einem Luftreiniger umfassend wenigstens eine Luftfördereinheit, einen Keiminaktivator mit wenigsten einer UV-Strahlungsquelle und wenigstens einem Luftauslass zur gerichteten Abgabe eines gereinigten Luftstroms in Richtung des Kopfes wenigstens eines Nutzers. Wesen der DE 20 2020 103 935 ist dabei, dass der durch den Luftauslass ausströmende Luftstrom am Tisch sitzende Personen durchschnittlicher Größe im Bereich von deren Gesicht, insbesondere im Bereich von Mund und Nase trifft. Auf diese Weise kann erreicht werden, dass die am Tisch sitzenden Personen mit keiminaktivierter Luft gerichtet angeblasen werden, wodurch ein, insbesondere durch Aerosole, bestehendes Infektionsrisiko mit in der Raumluft befindlichen Keimen deutlich reduziert wird.

DE 10 2020 121 130 A1 beschreibt eine Vorrichtung, die aerosolhaltiger Luft aus mindestens einem Absaugvolumen in einem unmittelbaren und lokal abgrenzbaren Umgebungsbereich über mindestens eine Ansaugöffnung lokal einsaugt, in der Vorrichtung sterilisiert und wieder in diesen Umgebungsbereich über mindestens eine Abluftöffnung wieder abgibt. Wesentlich ist, dass die Luft aus dem Volumen mit der lokalen Aerosolquelle angesaugt wird, wie beispielsweise in einem Restaurant zwischen an einzelnen Tischen sitzenden Personen.

Primär bei solchen Systemen ist, dass die zu schützende Person weitgehend ausschließlich die vom Gerät aufbereitete, desinfizierte Luft einatmet. Das damit verbundene direkte Anblasen der zu schützenden Person zumeist direkt im Kopfbereich mit der ausströmenden Luft wird jedoch häufig als sehr unangenehm empfunden. Weiterer Nachteil solcher Systeme ist, dass die kontaminierte Atemluft nicht direkt angesaugt und gereinigt wird, sondern sich im Raum weitgehend ungehindert ausbreiten kann. Damit steigt die Konzentration von Krankheitserregern im Raum.

Aus DE 10 2020 120 046 A1 ist ein Desinfektionsgerät bekannt, welches die kontaminierte Atemluft möglichst lokal absaugen soll. In DE 10 2020 120 046 A1 wird ein kompakt aufgebautes Tischgerät beschrieben, welches auf einem Tisch unmittelbar zwischen zwei oder auch mehr gegenübersitzenden oder stehenden Personen platziert wird und bei dem die Eintrittsöffnung für die angesaugte Luft auf etwa Kopfhöhe der jeweiligen Person und folglich (in etwa) auf "Sprechhöhe" angeordnet ist, um insbesondere die virenbelastete Luft in das Gerät einzusaugen. Die Eintrittsöffnung ist dabei oberhalb der Austrittöffnung im oberen Bereich des Gehäuses angeordnet. Mit der Fördereinrichtung wird Luft über die Eintrittsöffnung in das Gehäuse angesaugt, durch die Desinfektionseinrichtung gefördert und dabei desinfiziert und die desinfizierte Luft aus der Austrittsöffnung des Gehäuses ausgeblasen. Nachteil dabei ist, dass der Aufbau der Einsaugvorrichtung in Sprechhöhe zwischen den Personen an einem Tisch die Arbeitsatmosphäre erheblich beeinträchtigt. Deshalb wird in DE 10 2020 120 046 A1 dann als Lösung dafür optional die Möglichkeit vorgeschlagen, auf dem Gehäuse eine transparente Trennscheibe vorzusehen. Dabei geht die Erfindung von der Erkenntnis aus, dass sich das Tischgerät zwar mit der Eintrittsöffnung bis in Kopfhöhe und folglich bis in Sprechhöhe erstrecken soll, dass es jedoch die Personen, zwischen denen das Gerät angeordnet wird, in der Unterhaltung möglichst wenig stören soll.

Die Möglichkeit der Verwendung einer transparenten Trennscheibe zur Abgrenzung von Lufträumen und damit zur Verminderung der Infektionsgefahr zwischen Personen ist dabei grundsätzlich bekannt, hat jedoch nur eine geringe Akzeptanz und vermindert die Arbeitsatmosphäre und/oder Kundenfreundlichkeit erheblich. US 9 050 382 B2 schlägt deshalb eine Art Luftwand bzw. Luftbarriere, die zwischen am Tisch sitzenden Personen gebildet wird, durch die Ausströmluft vor. Dabei soll gezielt eine Luftströmung der ausgeblasenen Luft und damit eine Luftbarriere zwischen den sich gegenüber befindenden Personen so erzeugt werden, dass die Atemluft der Personen jeweils mit der Luftströmung nach oben abgeführt wird. Durch diese strömungstechnische Luftbarriere soll verhindert werden, dass die Ausatem- bzw. Sprechluft direkt von der gegenübersitzenden Person eingeatmet wird. Dabei wird jedoch die Ausatem- bzw. Sprechluft als Kontaminationsquelle vollständig mit der Luftströmung in den Raum gelenkt und kontaminiert diesen so entsprechend.

Es besteht daher Bedarf an einer Vorrichtung, die das Risiko einer Infektion durch das Einatmen von Aerosolen verringern kann, wenn zwei oder mehr Personen um einen Tisch, eine Theke oder ähnliches sitzen oder stehen. Insbesondere besteht ein Bedarf an einer Vorrichtung, welche die Benutzer visuell oder akustisch möglichst wenig stört und vorzugsweise kompakt ist. Weiterhin besteht Bedarf an einer Vorrichtung, welche kontaminierte Luft möglichst nicht in den Raum zurückführt und vorzugsweise eine geringe Keimbelastung im Raum aufrechterhält.

### Aufgabe der Erfindung

Es ist deshalb eine Aufgabe der vorliegenden Erfindung, eine Vorrichtung bereitzustellen, welche für die Verwendung auf oder mit Tischen, Tresen oder Theken geeignet ist, wobei die Vorrichtung vorzugsweise auch in ein Möbelstück integrierbar ist. Vorzugsweise bietet die Vorrichtung bei hoher Effizienz und geringem Energieverbrauch eine ausreichende Sicherheit vor infektiösen Aerosolen und gleichzeitig eine angenehme Atmosphäre für an einem Tisch, einem Tresen oder einer Theke befindliche Personen. Weiterhin ist es eine Aufgabe der Erfindung, ein System, Verwendung und ein Verfahren unter Verwendung der Vorrichtung bereitzustellen, welche die Mängel des Standes der Technik beseitigen.

### Zusammenfassung der Erfindung

Gelöst wird die Aufgabe durch die Merkmale der unabhängigen Ansprüche. Vorteilhafte Ausgestaltungen der Erfindung sind in den abhängigen Ansprüchen und in der Beschreibung offenbart.

In einem ersten Aspekt betrifft die Erfindung eine Vorrichtung zur Desinfektion von Luft. Die Vorrichtung umfasst eine Lufteinlasseinheit, eine Luftfördereinheit, eine Desinfektionseinheit und eine Luftauslasseinheit. Die Lufteinlasseinheit und die Luftfördereinheit sind für das Ansaugen von Ausatemluft von mindestens zwei Personen konfiguriert, die Luftfördereinheit ist für die Förderung der Ausatemluft von der Lufteinlasseinheit über die Desinfektionseinheit zu der Luftauslasseinheit konfiguriert und die Luftauslasseinheit ist für die Bildung einer Luftbarriere, vorzugsweise zwischen den mindestens zwei Personen, konfiguriert. Die Lufteinlasseinheit liegt in Umfangsrichtung um die Austrittsöffnung angeordnet vor, vorzugsweise so, dass mindestens ein Bereich der Luftauslasseinheit zwischen jeder der mindestens zwei Personen und der Lufteinlasseinheit positionierbar ist. Die Luftauslasseinheit ist mindestens teilweise von der Lufteinlasseinheit umgeben.

Durch die erfindungsgemäße Vorrichtung kann die Ausatem- bzw. Sprechluft als Aerosolquelle zumindest anteilig direkt angesaugt, in der erfindungsgemäßen Vorrichtung desinfiziert und die desinfizierte Luft aus der Luftauslasseinheit so ausgeblasen werden, dass die Luftströmung von einer Möbeloberfläche eine mindestens teilweise nach oben gerichtete Luftbarriere zwischen mehreren am Möbelstück (wie Tisch, Tresen oder Theke) befindlichen Personen ausbildet.

Durch die erfindungsgemäß ausgebildete Luftbarriere und die Gestaltung des angesaugten Luftvolumens durch die erfindungsgemäße Vorrichtung wird vorzugsweise erreicht, dass mindesten 60 %, vorzugsweise mindestens 70 %, bevorzugter mindestens 80 %, besonders bevorzugt mindestens 90 % bezogen auf die Anzahl der in der Ausatemluft der einen Person befindlichen Keime nicht in eine Raumzone einer anderen Person am Möbelstück gelangen, was das Ansteckungsrisiko durch luftübertragene Viruserkrankungen erheblich minimiert.

Im Sinne der Erfindung ist die "Raumzone" einer Person vorzugsweise ein Volumen, welches den Kopf und/oder mindestens die Nase und Mund der Person umgibt, ohne sich mit einem gleich großen Volumen um den Kopf und/oder Nase und Mund einer benachbarten Person zu überschneiden. Diese Raumzone umfasst vorzugsweise die Luft, die von der Person eingeatmet wird. Als Raumzone um eine Person an einem Möbelstück (wie Tisch, Tresen oder Theke) ist vorzugsweise ein Bereich definiert, der sich vom Kopf und Mund-Nasenbereich zum Einatmen der Atemluft bis zur Lufteinlasseinheit erstreckt. Vorzugsweise erstreckt sich die Raumzone mindestens 30 cm, besonders bevorzugt mindestens 40 cm vor dem Kopf und/oder dem Mund und Nase der Person. Vorzugsweise erstreckt sich die Raumzone auch mindestens 10 cm links, rechts, über und hinter dem Kopf bzw. Mund und Nase der Person. Die Raumzone ist vorzugsweise von unten durch eine Möbeloberfläche begrenzt, kann aber auch mindestens 40 cm in der Luft nach unten reichen.

Eine "Luftbarriere" im Sinne der Erfindung umfasst vorzugsweise einen oder mehrere Luftströme, die in eine erste Richtung strömen, so dass sie im Wesentlichen nicht von Luft aus anderen Richtungen durchströmt werden. Eine Luftbarriere (oder auch als Luftvorhang, Luftschranke, Luftschleier oder ähnliches bezeichnet) unterbindet vorzugsweise eine ungehinderte Luftströmung bzw. Luftaustausch zwischen zwei oder mehr definierten Luftbereichen eines Innenraums (Raumzonen) durch eine zielgerichtete Strömungsbewegung von Luft als Barriere. Vorzugsweise ist die Luftbarriere als Kegel oder als Keil ausgebildet. Inbegriffen ist dabei hier auch eine stumpfe Kegelform (Kegelstumpf), also ein Kegel mit parallel zur Grundfläche abgeschnittener Spitze oder einen stumpfen Keil (Keilstumpf), also ein Keil mit parallel zur Grundfläche abgeschnittener Keilspitze handeln. Vorzugsweise erzeugt die Luftbarriere einen Unterdruck in der Umgebungsluft, der die Luftströme von den Benutzern wegleitet. Vorzugsweise fließt ein Strom, der die Luftbarriere bildet, nach oben und annähernde Luftströme aus anderen Richtungen werden ebenfalls nach oben umgeleitet.

Indem eine Luftbarriere gebildet wird, anstatt die desinfizierte Luft direkt auf die Köpfe der Benutzer zu leiten, kann ein höheres Maß an Komfort erreicht werden. Darüber hinaus ist die Luftbarriere besonders praktisch für die Interaktion, da sie keine unerwünschten Reflexionen oder Flecken verursacht, wie man sie häufig bei Glas-Trennscheiben findet. Die Vorrichtung ist daher besonders förderlich für eine natürliche und ungehinderte Interaktion zwischen Personen.

Durch die Anordnung der Eintrittsöffnungen um den Umfang der Austrittsöffnung (auch "periphere Anordnung" im Sinne der Erfindung) sind diese besonders geeignet, die Atemluft der Benutzer direkt abzusaugen, welche die kritischste Quelle von Aerosolen in einem Raum darstellt. Indem diese Luft abgesaugt wird, bevor sie im Raum verteilt wird, ist die Vorrichtung besonders effizient darin, die Verbreitung solcher Aerosole zu verhindern. Außerdem ermöglicht die zentrale Positionierung der Austrittsöffnung die Schaffung einer Luftbarriere, die den Luftraum von mindestens zwei Personen wesentlich voneinander trennt. Dies verhindert die Vermischung der von den beiden Personen ausgeatmeten Aerosole. Zusätzlich ist es von Vorteil, dass die Luft, die zur Bildung der Luftbarriere ausgestoßen wird, frisch desinfizierte Luft ist. Damit überwindet die Vorrichtung den Nachteil der nach dem Stand der Technik üblichen Lösungen, bei denen aerosolhaltige Luft in denselben Raum zurückgeführt wird, wodurch sich die Gesamtkeimbelastung darin mit der Zeit erhöht.

Im Sinne der Erfindung ist eine "Umfangsrichtung" vorzugsweise eine Richtung, die sich von einem Mittelpunkt eines Gegenstandes zu einem beliebigen Punkt außerhalb des Gegenstandes in einer Ebene erstreckt, wobei die Ebene vorzugsweise horizontal ist und vorzugsweise einer Oberfläche eines Möbelstücks wie einem Tisch entspricht. Peripher (oder in "Umfangsrichtung") angeordnete Gegenstände müssen hierbei nicht selbst in der gleichen horizontalen Ebene wie ein zentraler Gegenstand liegen, sondern können über oder unter dieser Ebene positioniert sein. Insbesondere wenn die Anordnung von oben oder unten betrachtet wird, sollte die zentrale Positionierung eines Gegenstands zwischen oder teilweise umgeben von peripheren Gegenständen vorzugsweise erkennbar sein.

Eine Anordnung der Lufteinlasseinheit in Umfangsrichtung um eine Luftauslasseinheit bedeutet vorzugsweise, dass sich die Lufteinlasseinheit in mindestens zwei von der Mitte der Luftauslasseinheit ausgehenden Richtungen um die Luftauslasseinheit positioniert ist. Zum Beispiel kann die Lufteinlasseinheit im Wesentlichen den gesamten Umfang einer Austrittsöffnung umschließen. Dies kann durch eine durchgehende schleifenförmige Lufteinlasseinheit erreicht werden. Alternativ kann die Lufteinlasseinheit bogen- oder hufeisenförmig sein und zumindest einen Teil des Umfangs der Austrittsöffnung umschließen, was beispielsweise einem Öffnungswinkel von mindestens 45°, vorzugsweise mindestens 60°, noch bevorzugter mindestens 120° und noch bevorzugter mindestens 180° entspricht. In einem weiteren bevorzugten Beispiel kann die Luftauslasseinheit zwischen zwei Eintrittsöffnungen einer Lufteinlasseinheit angeordnet sein. Die Luftauslasseinheit kann auch von drei, vier oder mehr Eintrittsöffnungen umgeben sein.

Im Sinne der Erfindung ist eine "Lufteinlasseinheit" vorzugsweise eine Konstruktion, die so konfiguriert ist, dass sie Luft in die Vorrichtung einströmen lässt oder aktiv ansaugt. Die Lufteinlasseinheit kann eine oder mehrere Eintrittsöffnungen umfassen, die ihrerseits eine oder mehrere Stabilisierungsrippen, Filter, Gitter, Blenden und/oder Ventilatoren umfassen können. Die Eintrittsöffnungen sind vorzugsweise getrennt, können aber auch nebeneinander liegen. Die Eintrittsöffnungen können nach oben gerichtet sein, eine Ebene mit einer Möbeloberfläche bilden, aus einer Möbeloberfläche herausragen und/oder geneigt sein, insbesondere in Richtung der mindestens zwei Personen. Die Lufteinlasseinheit hat vorzugsweise eine Form, die sich aus der einzigen Eintrittsöffnung oder aus der Anordnung der mehreren Eintrittsöffnungen ergibt, wie z.B. ein Ring, ein Doppelschlitz, ein Bogen, ein Quadrat, ein Stern oder Ähnliches.

Im Sinne der Erfindung ist eine "Luftauslasseinheit" vorzugsweise eine Konstruktion, die so konfiguriert ist, dass sie Luft aus der Vorrichtung ausströmen lässt oder aktiv ausstößt. Die Luftauslasseinheit kann eine oder mehrere Austrittsöffnungen umfassen, die ihrerseits eine oder mehrere Stabilisierungsrippen, Filter, Gitter, Blenden und/oder Ventilatoren umfassen können. Die Austrittsöffnungen können vorzugsweise angrenzend aneinander liegen. Die Austrittsöffnungen können nach oben gerichtet sein, eine Ebene mit einer Möbeloberfläche bilden, aus einer Möbeloberfläche herausragen und/oder geneigt sein, insbesondere in einer diagonalen Richtung zwischen einer vertikalen und einer seitlichen Richtung, welche vorzugsweise die mindestens zwei Personen voneinander trennt. Die Luftauslasseinheit hat vorzugsweise eine Form, die sich aus der einzigen Austrittsöffnung oder aus der Anordnung der mehreren Austrittsöffnungen ergibt, wie z.B. ein Kreis, ein Quadrat, ein Stern oder Ähnliches.

Bei Ausführungsformen der Luftauslasseinheit mit mehreren Austrittsöffnungen ist es bevorzugt, dass diese Austrittsöffnungen gebündelt sind. Um die Austrittsöffnungen herum ist die Lufteinlasseinheit in Umfangsrichtung angeordnet, beispielsweise in Form eines Rings oder eines Bogens. Es kann auch bevorzugt sein, dass eine oder mehrere Austrittsöffnungen der Lufteinlasseinheit eine Sternform bilden, zum Beispiel einen drei-, vier- oder fünfspitzigen Stern. Die Spitzen des Sterns können bevorzugt im Wesentlichen länglich gestaltet sein und können so konfiguriert sein, dass sie die Lufträume von Benutzern, z.B. an einem runden Tisch, trennen. In diesem Fall bedeutet eine umfängliche Anordnung (oder Anordnung in Umfangsrichtung) der einen oder mehreren Eintrittsöffnungen die Platzierung der Eintrittsöffnungen zwischen den Speichen des Sterns. Auf diese Weise umgeben sie den Mittelpunkt der Luftauslasseinheit.

Unter Ausatemluft ist vorzugsweise jegliche Art über Mund und/oder Nase ausgestoßene Luft einer Person mit den darin enthaltenden Flüssigkeitströpfchen, insbesondere den Aerosolen zu verstehen, so wie sie beispielsweise beim Atmen, Sprechen, Singen, Husten oder Niesen entsteht. Befinden sich Krankheitserreger beispielsweise in den Atemwegen der ausstoßenden Personen, so können die mit der Ausatemluft ausgestoßenen Flüssigkeitströpfchen auch diverse Krankheitserreger enthalten.

Im Sinne der Erfindung ist eine "Luftfördereinheit" vorzugsweise eine Konstruktion, die so konfiguriert ist, dass sie Luft von der Lufteinlasseinheit zur Luftauslasseinheit transportiert. Die Luftfördereinheit kann ein Ventilator und/oder eine Pumpe umfassen.

Im Sinne der Erfindung ist eine "Desinfektionseinheit" vorzugsweise eine Konstruktion, welche Mittel zur Reduzierung der Anzahl von aktiven Keimen in einem Luftstrom umfasst. Dies kann chemische, physikalische oder elektromagnetische Mittel umfassen. Die Desinfektionseinheit kann beispielsweise eine beliebige Anzahl von Filtern, Absorptionsmitteln, Adsorptionsmitteln, Heizungen, Kühlungen, desinfizierenden Materialien und/oder UV-Strahlern umfassen, wobei mindestens ein UV-Strahler bevorzugt wird.

Vorzugsweise sind die Lufteinlasseinheit, Desinfektionseinheit und Luftauslasseinheit durch einen oder mehrere Kanäle miteinander verbunden. Die Kanäle von mehreren Eintrittsöffnungen können insbesondere stromaufwärts der Desinfektionseinheit zusammengeführt werden. Der Kanal kann optional stromabwärts der Desinfektionseinheit abzweigen, um desinfizierte Luft zu mehreren Austrittsöffnungen zu leiten.

Die Vorrichtung ist vorzugsweise für die Verwendung auf die Oberfläche eines Möbelstücks ausgestaltet, insbesondere auf einem Tisch, einer Theke oder einem Tresen.

Vorzugsweise wird die Ausatemluft von sich an dem Möbelstück (wie Tisch, Theke oder Tresen) befindenden Personen zumindest teilweise angesaugt werden.

Im Sinne der Erfindung ist ein "Möbelstück" vorzugsweise eine Vorrichtung, die eine im Wesentlichen horizontale Oberfläche definiert, die vom Boden abgehoben ist. Die horizontale Oberfläche ist vorzugsweise mindestens 70 cm, besonders bevorzugt mindestens 1 m vom Boden entfernt, wenn das Möbelstück in seiner normalen Ausrichtung auf den Boden gestellt wird. Ein solches Möbelstück ist vorzugsweise so gestaltet, dass es von mehreren Personen benutzt werden kann, die um das Möbelstück herum oder auf einer Seite des Möbelstücks angeordnet sein können. Es kann besonders bevorzugt sein, dass das Möbelstück mindestens zwei Personen trennt, indem es sich insbesondere während der Benutzung zwischen ihnen befindet. Vorzugsweise ist die horizontale Oberfläche des Möbelstücks so gestaltet, dass sie bei normaler Benutzung mindestens die Hüfthöhe der Benutzer erreicht. Bei dem Möbelstück kann es sich beispielsweise um einen Tisch wie einen Esstisch in einem Restaurant handeln. Das Möbelstück kann auch ein Schreibtisch sein, z. B. ein Schreibtisch für mehrere Personen in einem gemeinsam genutzten Büroraum. Das Möbelstück kann auch eine Theke oder ein Tresen sein, wie sie typischerweise in Bars, Hotels und Geschäften verwendet werden, wo ein Benutzer (z. B. ein Rezeptionist) sitzen und ein anderer (z. B. ein Kunde) während der Benutzung des Möbelstücks stehen kann. Das Möbelstück kann auch eine Arbeitsstation sein, zum Beispiel in einer Produktionsstätte.

Von dem Begriff Tisch sind vorzugsweise jegliche Formen wie rund, oval, eckig und jegliche Größe umfasst, an denen Personen sitzen oder stehen können und befinden sich beispielsweise in Arbeits-, Besprechungs-, Tagungs-, Konferenz, Schul(ungs)räumen. Als Theke ist die tischähnliche Trennung zwischen Personal und Kunden bevorzugt in Gaststätten, Kneipen oder Bars zu verstehen, an der beispielsweise die Warenübergabe und/oder die Bezahlung erfolgt. Unter Tresen ist bevorzugt ein Verkaufstisch beispielsweise in einem Geschäft oder Serviertisch wie beispielsweise in Gaststätten, Kneipen oder Bars zu verstehen. Die Begriffe Tresen und Theke können bevorzugt auch synonym verwendet werden und umfassen auch die manchmal verwendeten Unterbegriffe wie Schanktisch, Ladentisch oder Ladentheke. Die räumliche Abtrennung mithilfe eines Möbelstücks von beispielsweise Kassenbereichen zwischen Kassierer und Kunden in Geschäften ist dabei hier vom Begriff Tresen umfasst.

Kritisch im Sinne eines Ansteckungsrisikos durch infektiöse Personen ist, dass virusbehaftete Aerosole in der Ausatemluft durch andere Personen eingeatmet werden. Dabei sinkt das Ansteckungsrisiko signifikant mit zunehmender Entfernung, die die Ausatemluft mit den virusbehafteten Aerosolen zurücklegen muss. Da sich beispielsweise in bestimmten Arbeits- und Gesprächssituation Personen in dem Abstandsbereich befinden müssen oder wollen, die für eine Virusansteckung bei direkter Zuströmung der Ausatemluft besonders kritisch sind (zum Beispiel Abstand kleiner 2 m), kann durch Maßnahmen zur Unterbindung dieser direkten Zuströmung der Ausatemluft zur anderen Person das Ansteckungsrisiko vermindert werden.

So wird durch die Ausbildung einer Luftbarriere durch eine gerichtete Strömung der von der Vorrichtung ausgestoßenen und desinfizierten Luft zwischen sich am Möbelstück befindenden Personen verhindert, dass die in der Ausatemluft der einen Person befindenden Aerosole direkt zu einer anderen Person am Tisch gelangen. In diesem Sinne ist eine direkte Strömung vorzugsweise eine Strömung, welche in einer ungehinderten Strömungsgrundrichtung von dem Mund-Nasenbereich einer Person zu einer anderen Person am Tisch verläuft. Die Strömung kann hierbei auch turbulent sein.

Vorzugsweise wird eine gerichtete Strömung zwischen den voneinander zu trennenden Luftbereichen (Raumzone) durch die Vorrichtung ausgebildet. Eine Luftströmung quer zu dieser gebildeten Luftbarriere wird durch die Luftströmung der Luftbarriere abgelenkt, sofern die Luftströmung der Luftbarriere größer ist. Dadurch kann wirksam eine direkte Luftströmung zwischen den abzutrennenden Luftbereichen (Raumzonen) und insbesondere damit der direkte Transport der ausgestoßenen Aerosole zu einer anderen Person am Möbelstück verhindert werden.

Die geometrische Form der Luftbarriere ist dabei erfindungsgemäß so gestaltet, dass die Luftbarriere die zu separierenden Raumzonen abtrennt. Das heißt insbesondere, dass das direkte Zuströmen ausgestoßener Aerosole mit der Atemluft einer Person zu einer anderen Person am Möbelstück vermindert wird. Dabei kann die Luftbarriere beispielsweise in Form einer Wand, Säule, Keil oder Kegel ausgebildet sein. Anders als bei materiellen Barrieren wie Glasscheiben, hat eine Luftbarriere jedoch keine klar abgrenzbare Form an den Randbereichen, da die Strömungsgeschwindigkeit der ausströmenden Luft an der Auslassvorrichtung die höchste Geschwindigkeit hat und mit der Entfernung von der Auslassvorrichtung abnimmt. Dies hat den Vorteil, dass ein kontraproduktiver Coanda-Effekt, welcher oft an den Rändern einerfesten Barriere auftritt, vermieden werden kann. Da dieser Effekt dazu führen kann, dass Fluidströme (in diesem Fall ausgeatmete Luft) "um eine Ecke" strömen, ist es besonders vorteilhaft, dies zu vermeiden, indem eine Luftbarriere statt einer festen Barriere verwendet wird.

In einer bevorzugten Ausführungsform der Erfindung hat die durch die Vorrichtung erzeugbare Luftbarriere eine vertikale konische Form mit einer nach unten gerichteten Spitze, eine vertikale keilartige Form oder eine Kombination davon.

Eine vertikale Erstreckung der Luftbarriere, insbesondere nach oben, kann Personen, die an einem Möbelstück sitzen, besonders effektiv trennen und eine unerwünschte Vermischung der Luft aus ihren jeweiligen Raumzonen verhindern. Da die meisten Menschen an einem Möbelstück sitzen oder stehen, verlässt der Atem die Nase oder den Mund in einer im Wesentlichen horizontalen Richtung. Ein solcher im Wesentlichen horizontaler Luftstrom wird sehr effektiv durch einen vertikalen Luftstrom umgelenkt, so dass er die Luftbarriere nicht überqueren kann. Eine ungewollte Vermischung der Luft wird so unterbunden. Verschiedene Formen der im Wesentlichen vertikalen Luftbarriere können in verschiedenen Situationen von Vorteil sein. Wenn sich beispielsweise zwei Personen gegenübersitzen, kann eine Luftbarriere in Form einer vertikalen Ebene sie effektiv voneinander trennen, ohne Unbehagen zu verursachen, da die desinfizierte Luft nicht direkt auf ihre Gesichter gerichtet ist. Bei drei oder mehr Personen, die in einem nicht orthogonalen Winkel zueinander sitzen, z.B. an einem runden Tisch, kann eine kegelförmige Luftbarriere vorteilhafter sein, da das breitere Volumen der Luftbarriere einen ausreichenden Unterdruck bietet, um die ausgeatmete Luft einer Person von der Raumzone anderer Personen wegzulenken.

In einer bevorzugten Ausführungsform der Erfindung ist die Luftauslasseinheit für das Ausblasen von desinfizierter Luft nach oben orientiert, insbesondere wenn die Vorrichtung auf eine Oberfläche eines Möbelstücks positioniert vorliegt oder darin integriert ist. Vorzugsweise ist die mindestens eine Austrittsöffnung der Luftauslasseinheit so ausgebildet, dass der Luftstrom mit der desinfizierten Luft von der Möbeloberfläche betrachtet nach oben gerichtet ist und sich durch die ausströmende Luft eine Luftbarriere zwischen den am Möbelstück sitzenden Personen ausbildet.

Im Sinne der Erfindung ist ein nach oben gerichteter Luftstrom vorzugsweise ein mit einer durchschnittlichen Strömungsrichtung, die einen Winkel von 90 ° zu einer horizontalen Oberfläche bildet. Es kann jedoch auch bevorzugt sein, dass die durchschnittliche Strömungsrichtung des Stroms einen Winkel zwischen 60 - 120°, insbesondere zwischen 70 - 100° zur horizontalen Oberfläche bildet. Bei einer solchen Strömungsrichtung - im Gegensatz zum Stand der Technik - kann die ausgestoßene Luft eine Person effektiv von einer anderen trennen, ohne direkt auf das Gesicht einer Person zu treffen und Kälte oder Unbehagen zu verursachen. Zusätzlich kann die Luftbarriere in Kopfhöhe der Benutzer entsprechend breit sein, so dass ein wirksamer Schutz vor Luftvermischung gewährleistet ist.

Vorzugsweise werden die Raumzonen um Personen an einem Möbelstück (wie Tisch, Tresen oder Theke) durch die Luftbarriere getrennt. Eine Raumzone umfasst vorzugsweise mindestens den Bereich der ausgestoßenen Ausatemluft, der zumindest anteilig eingesaugt werden soll.

Da die Strömungsgeschwindigkeit der erzeugten Luftbarriere vorzugsweise an der Austrittsöffnung am höchsten ist, ist diese Austrittsöffnung vorzugweise im oder auf einem Möbelstück integriert und die erzeugte Luftströmung von der Möbeloberfläche aus betrachtet nach oben gerichtet. Die nach oben gerichtete Luftströmung hat zudem den Vorteil, dass Luft der Raumzone nach oben von der Person am Möbelstück weggeführt wird.

Würde eine solche Austrittsöffnung beispielsweise an der Decke platziert werden und die resultierende Luftströmung von dort betrachtet nach unten gerichtet sein, würde die abgelenkte oder mitgeführte Luft der Raumzone nach unten in den Möbelbereich transportiert, dort aufprallen und umgelenkt werden. Da in diesem Bereich dann die Luftbarrierenwirkung aufgrund der Entfernung vom Luftauslass gering ist, kann die Luft aus der einen Raumzone sehr viel leichter und auf wesentlich kürzerem Weg in eine andere Raumzone eindringen, was die Ansteckungsgefahr entsprechend erhöht.

Die Luftauslasseinheit wird vorzugsweise so ausgebildet, dass die bevorzugte Form der Luftbarriere resultiert. Dies wird beispielsweise durch die Einstellung des Ausblaswinkels, des Volumenstroms und der Geschwindigkeit erreicht. Der Ausblaswinkel beeinflusst beispielsweise die Strömungsrichtung. Der Volumenstrom und die Ausblasgeschwindigkeit beeinflussen beispielsweise die Stärke der Barrierewirkung und die Ausdehnung der Luftbarriere.

In einer weiteren bevorzugten Ausführungsform der Erfindung umfasst die Lufteinlasseinheit mehrere Eintrittsöffnungen, wobei vorzugsweise eine Eintrittsöffnung pro Person vorgesehen ist. Eine solche Vorrichtung kann besonders effektiv sein, da die Eintrittsöffnungen optimal positioniert und/oder geneigt werden können, um die Ausatemluft von Personen aufzunehmen, die an bekannten Positionen an einem Möbelstück sitzen oder stehen. Zum Beispiel können die Eintrittsöffnungen in einem Winkel von etwa 30° - 60° zur Möbelstückoberfläche und zur jeweiligen Person hingeneigt sein. Auf diese Weise umfasst die angesaugte Luft einen größeren Anteil an ausgeatmeter Luft und das unerwünschte Ansaugen von frisch desinfizierter Luft kann vermieden werden. Außerdem kann eine solche Vorrichtung besonders energieeffizient gestaltet werden, indem die einzelnen Eintrittsöffnungen ungenutzt bleiben können, wenn nicht erkannt wird, dass eine Person an der entsprechenden Stelle sitzt oder steht. Zum Beispiel kann ein Bewegungssensor eine Eintrittsöffnung automatisch außer Betrieb setzen, wenn für eine vorher festgelegte Zeitspanne, beispielsweise zehn Minuten, keine Bewegung erkannt wird. Die Eintrittsöffnung kann außer Betrieb gesetzt werden, indem eine Abdeckung geschlossen wird, ein Gitter geschlossen wird, ein entsprechender Ventilator gestoppt wird oder die Eintrittsöffnung anderweitig blockiert wird.

In einer bevorzugten Ausführungsform der Erfindung sind die Fördereinrichtung und die Luftauslasseinheit so konfiguriert, dass die Strömungsgeschwindigkeit der ausgestoßenen desinfizierten Luft größer als eine Strömungsgeschwindigkeit ausgeatmeter Luft ist. Insbesondere sollte die Strömungsgeschwindigkeit der Luft aus der Luftauslasseinheit die Strömungsgeschwindigkeit der ausgeatmeten Luft übersteigen, wenn diese ausgeatmete Luft die Luftauslasseinheit oder Luftbarriere erreicht hat. Auf diese Weise kann die ausgeatmete Luft effektiv durch die Luftbarriere umgelenkt werden. Vorzugsweise beträgt die Strömungsgeschwindigkeit der Luft aus der Luftauslasseinheit mindestens 0,2 m/s, bevorzugter mindestens 0,5 m/s, noch bevorzugter mindestens 1 m/s.

In einer bevorzugten Ausführungsform der Erfindung sind die Luftfördereinheit und die Luftauslasseinheit für eine Strömungsgeschwindigkeit aus der Luftauslasseinheit im Bereich von 0,2 - 2,5 m/s, vorzugsweise 0,5 - 1,5 m/s, noch bevorzugter 1,0 - 1,5 m/s ausgelegt. Es wurde überraschenderweise festgestellt, dass diese Strömungsgeschwindigkeiten ein ausgezeichnetes Gleichgewicht zwischen der effektiven Umleitung der ausgeatmeten Luft, dem Benutzerkomfort und dem Geräuschpegel bieten.

In einer bevorzugten Ausführungsform sind die Luftfördereinheit und Luftauslasseinheit für eine Strömungsgeschwindigkeit direkt an der Austrittsöffnung im Bereich von 0,5 m/s bis 1,5 m/s konfiguriert. Festgestellt wurde dabei, dass in diesem Geschwindigkeitsbereich jeweils eine geeignete Luftbarriere für sehr unterschiedliche Anwendungsfälle und Arbeitssituationen erzeugt werden kann.

In einer besonders bevorzugten Ausführungsform liegt die Strömungsgeschwindigkeit direkt an der Austrittsöffnung im Bereich von 0,2 m/s bis 1,0 m/s. Bei diesen vergleichsweise geringen Strömungsgeschwindigkeiten resultiert typischerweise eine geringere Geräuschemission, was eine angenehme Arbeits- und Gesprächsatmosphären begünstigt, dennoch kann eine ausreichende Barrierewirkung insbesondere an Arbeits- und Besprechungstischen bei sitzenden Personen erreicht werden.

In einer anderen, ebenfalls besonders bevorzugten Ausführungsform liegt die Strömungsgeschwindigkeit direkt an der Austrittsöffnung im Bereich von 1,0 m/s bis 2,5 m/s. Bei diesen vergleichsweise hohen Strömungsgeschwindigkeiten ist die Wirkung der Luftbarriere entsprechend größer, weshalb sich diese Ausführungsform beispielsweise für Anwendungssituationen eignet, bei denen eine oder mehrere Personen am Tisch, Tresen oder der Theke sitzen und stehen.

In einer bevorzugten Ausführungsform der Erfindung sind die Lufteinlasseinheit und/oder die Luftauslasseinheit auf Höhe der Möbeloberfläche angeordnet und können in der Möbeloberfläche integriert sein. Dazu kann beispielsweise eine Lufteinlass- und/oder Luftauslasseinheit fest mit der Oberfläche verbunden oder in dieser eingesteckt sein. Auch können entsprechend konstruierte Öffnungen in der Möbeloberfläche selbst bereits die Lufteinlass- und/oder Luftauslasseinheit für die Luft in bzw. aus der Vorrichtung darstellen. Diese Ausführungsform hat dabei beispielsweise den Vorteil, dass die Lufteinlass- und/oder Luftauslasseinheit keine störenden Aufbauten darstellen.

In einer ebenfalls bevorzugten Ausführungsform der Erfindung ist die Luftauslasseinheit in dergleichen oder einer höheren horizontalen Ebene als die Lufteinlasseinheit, insbesondere wenn die Vorrichtung auf die Oberfläche des Möbelstücks positioniert vorliegt oder darin integriert ist. Auf diese Weise kann die Luftbarriere besonders effektiv hergestellt werden, ohne dass die ausgestoßene desinfizierte Luft sofort wieder angesaugt wird. Stattdessen wird die ausgeatmete Atemluft effizient abgesaugt und desinfiziert.

In einer besonders bevorzugten Ausführungsform der Erfindung ist die Luftauslasseinheit nicht mehr als 50 cm, vorzugsweise nicht mehr als 30 cm, noch bevorzugter nicht mehr als 15 cm höher als die Oberfläche des Möbelstücks, insbesondere wenn die Vorrichtung auf die Oberfläche des Möbelstücks positioniert oder darin integriert ist. Hierbei können die Lufteinlasseinheit und Luftauslasseinheit unterschiedlich hoch sein. Auf diese Weise stellt die Vorrichtung keine wesentliche visuelle Barriere zwischen zwei Personen dar und ist besonders kompakt. Weiterhin wird ein Arbeitsbereich des Möbelstücks im Wesentlichen nicht beeinträchtigt. Zudem kann bei dieser Positionierung der Eintrittsöffnung die ausgeatmete Luft besonders effizient eingesaugt werden. Aufgrund der begrenzten Höhe der Eintrittsöffnung können fallende Tröpfchen - welche besonders infektiös sein können - effektiv zur Eintrittsöffnung umgeleitet werden. Hierdurch kann die Kontamination nicht nur der Raumluft, sondern auch der Möbelstückoberfläche reduziert werden. Gleichzeitig können effektive Luftbarrieren aus dieser Position errichtet werden, welche ohne exzessiven Geräuschpegel bis zu den Köpfen der Personen reichen kann, die das Möbelstück benutzen. Diese können insbesondere erreicht werden, wenn die Luftauslasseinheit bis zu 50 cm höher als die Lufteinlasseinheit liegt. Ist dabei beispielsweise die Luftauslasseinheit in der horizontalen Oberfläche eines Möbelstückes (wie in der Platte eines Tischs) integriert und liegt die Lufteinlasseinheit im Bereich bis zu 50 cm darüber, kann insbesondere bei eng aneinander liegenden Luftaus- und Lufteinlasseinheit die gegenseitige Störung oder Vermischung von Ansaugluftströmung und Ausblasluftströmung vorzugsweise verringert oder eliminiert werden.

Die sich an einem Möbelstück befindenden Personen können beispielsweise sitzen oder stehen und unterschiedliche Tätigkeiten weitgehend allein (zum Beispiel Schreiben, Lesen) oder in Interaktion miteinander (beispielsweise Gespräch) durchführen.

Vorzugsweise wird die Ausatemluft von sich an einem Möbelstück befindenden Personen über mindestens eine Eintrittsöffnung zumindest teilweise angesaugt.

In einer bevorzugten Ausführungsform der Erfindung wird die ausgeatmete Ausatemluft zumindest teilweise von der Vorrichtung angesaugt. Dies bedeutet vorzugsweise, dass mindestens 10 Vol.-%, vorzugsweise mindestens 30 Vol.-%, besonders bevorzugt mindestens 50 Vol-% und noch bevorzugter mindestens 70 Vol-% der Ausatemluft von der Vorrichtung angesaugt und/oder eingesaugt wird. Dabei bezieht sich diese und die folgenden Vol%-Angabe zur angesaugten Ausatemluft vorzugsweise auf die gesamte Ausatemluft aller sich am Möbelstück befindender Personen in Summe. Dabei befindet sich eine Person vorzugsweise dann am Möbelstück, wenn der Mund- und Nasenbereich der Person nicht weiter als 0,7 m von der Eintrittsöffnung der erfindungsgemäßen Vorrichtung für die angesaugte Luft entfernt ist.

Um mindestens 10 Vol.-% der Ausatemluft durch die Vorrichtung an- und einzusaugen, wird das Ansaugvolumen der Vorrichtung vorzugsweise so dimensioniert, dass sich eine vom Mund- und Nasenbereich der sich am Tisch befindenden Personen in Richtung der Eintrittsöffnung gerichtete dominierende Strömung ausbildet. Die eine Eintrittsöffnung ist oder die mehreren Eintrittsöffnungen der Lufteinlasseinheit sind vorzugsweise dazu so konstruiert, so positioniert oder so positionierbar, dass sie durch das Ansaugvolumen eine derartige dominierende Strömung ausbilden. In einer bevorzugten Ausführungsform ist die eine Eintrittsöffnung oder sind die mehreren Eintrittsöffnungen so ausgerichtet, dass jeweils eine Gerade zwischen dem Mund- und Nasenbereich der sich am Tisch, Theke oder Tresen befindenden Personen und der Eintrittsöffnung gebildet werden kann. Die Menge an eingesaugtem Volumen der Ausatemluft kann an der Vorrichtung beispielsweise durch Regulierung der Drehzahl eines Abluftventilators oder durch Einstellung an mindestens einem mechanischen Volumenstromregler (wie Volumenstrommessdüse mit Einstellklappe, Drosselklappen), die in der Vorrichtung integriert sein können, erfolgen.

In einer weiteren bevorzugten Ausführungsform werden mehr als 70 Vol.-% der Ausatemluft von der Vorrichtung angesaugt. Durch diesen hohen Anteil an eingesaugter Luft wird folglich auch ein entsprechend hoher Anteil an ausgestoßenen Aerosolen, die beispielsweise potentiell Viren enthalten können, in das Gerät eingesaugt und kann dort einer Desinfektion unterzogen werden. Mit dieser Ausführungsform wird somit eine besonders hohe Schutzwirkung erzielt. In einer anderen, ebenfalls bevorzugten Ausführungsform werden zwischen 30-70 Vol.-% der Ausatemluft von der Vorrichtung angesaugt. In diesem Bereich ist die Schutzwirkung zwar etwas geringer, dafür kann die Einsaugleistung der Vorrichtung reduziert werden, wodurch eine besonders niedrige Geräuschemission erreicht werden kann.

Bei mehreren Eintrittsöffnungen kann die Menge eingesaugter Luft über diese unterschiedlichen Eintrittsöffnungen unterschiedlich dimensioniert sein. So kann beispielsweise je nach Anwendungssituation die gewünschte Menge an Ausatemluft der sich am Tisch befindenden Personen tatsächlich eingesaugt werden. Beispielsweise können die Eintrittsöffnungen für eine Kombination von stehenden und sitzenden Personen mit unterschiedlichen Entfernungen bezogen auf die Lufteintrittsöffnung am Möbelstück ausgelegt sein.

Die Einstellung unterschiedlicher Mengen eingesaugter Luft an den unterschiedlichen Eintrittsöffnungen kann beispielsweise durch unterschiedliche Ansaugwirkungen durch jeweils angepasste Luftstromgeschwindigkeiten und -volumenströme an den Eintrittsöffnungen erfolgen. Dazu können die Eintrittsöffnungen an der Vorrichtung zum Beispiel bereits konstruktiv dahingehend gestaltet (größere Öffnungsvolumen für größere Ansaugvolumen) oder einstellbar (beispielsweise durch Anpassung der Öffnung durch Schiebe- oder Trennelemente) sein.

Zur Steuerung der eingesaugten Luftmenge kann die erfindungsgemäße Vorrichtung mindestens einen Sensor zum Erfassen der Anwesenheit einer Person und/oder der Entfernung zwischen dem Mund-Nasenbereich einer sich am Möbelstück befindlichen Person von der Lufteinlassöffnung. Dadurch kann zum Beispiel in Zusammenspiel mit einer Steuervorrichtung ein Regelkreis eingerichtet werden, durch welchen die eingesaugte Luftmenge so angepasst wird, dass genau die gewünschte Menge an Ausatemluft der Person am Möbelstück eingesaugt wird. Dazu kann die Vorrichtung beispielsweise im Bereich der Eintrittsöffnung eine durch den Regelkreis steuerbare Drosselklappen aufweisen, die den Volumenstrom einstellt. Durch die Steuerung der eingesaugten Luftmenge wird dabei beispielsweise die Geräuschentwicklung für die jeweilige Nutzungssituation durch die Reduzierung auf die notwendige Leistung der Luftfördereinheit minimiert.

Vorzugsweise wird die angesaugte Luft durch die Desinfektionseinrichtung gefördert.

Dazu weist die erfindungsgemäße Desinfektionseinrichtung mindestens einen Durchströmungskanal durch das Gerät auf, wobei der Querschnitt des Durchströmungskanals an verschiedenen Stellen nicht zwingend einheitlich konstant sein muss. Eine vorzugsweise geradlinige Ausgestaltung wird für einen geringen Strömungswiderstands vorgeschlagen, die aber auch Strömungsumlenkungsbereiche insbesondere aufgrund der Geometrie der Desinfektionseinheit aufweisen kann.

Auch können mindestens ein Durchströmungskanal in der erfindungsgemäßen Vorrichtung aufgeteilt oder mehrere, gegebenenfalls zuvor geteilte Strömungskanäle beispielsweise für eine kompaktere Gerätebauweise oder eine geringere Geräuschemission durch die Luftströmung in der Vorrichtung zusammengeführt werden.

Zur Förderung der angesaugten Luft durch die erfindungsgemäße Vorrichtung weist die Vorrichtung mindestens eine Luftfördereinheit auf, die beispielsweise als Lüfter, insbesondere als Axiallüfter oder Radiallüfter ausgebildet sein kann.

Vorzugsweise wird die eingesaugte Luft in der erfindungsgemäßen Vorrichtung desinfiziert.

Als Desinfektion wird vorzugsweise die Schädigung des Erbguts von Keimen durch UV-Strahlung verstanden. Dabei werden durch eine UV-Strahlungsquelle in RNA bzw. DNA Schäden hervorgerufen, sodass diese dann nicht mehr vermehrungsfähig, heißt inaktiviert und somit unschädlich gemacht sind.

Die Desinfektion inaktiviert vorzugsweise mindestens 90 % bezogen auf die Anzahl der im eingesaugten Volumenstrom befindlichen Keime. Dies entspricht einer log-Stufe. Zur Definition der Wirksamkeit der Desinfektion werden häufig auch die sogenannten log-Stufen verwendet. Die log-Stufe ist dabei die Maßeinheit, welche die Reduktion der Anzahl die lebensfähigen Mikroorganismen, hier Keime, definiert. Dieses berechnet sich, anhand der gemessenen Keimreduktion nach Desinfektion. Eine log-Stufe beschreibt dabei jeweils die Reduktion um eine Zehner-Potenz. Somit bedeutet 1-log-Stufe eine Reduktion der Keime um 90 %.

Überraschenderweise wurde festgestellt, dass bereits diese vergleichsweise geringe Desinfektion durch das erfindungsgemäße Funktionskonzept ausreichend Schutz für die Personen am Möbelstück bietet. Das erfindungsgemäße Funktionskonzept basierend dabei auf der Kombination aus Desinfektion der eingesaugten Ausatemluft als Aerosolquelle und der Ausbildung einer Luftbarriere zwischen den am Möbelstück sitzenden Personen durch eine gezielte Luftströmung, wodurch keim-inaktivierte Raumzonen für die Personen zum jeweiligen Ein- und Ausatmen geschaffen bzw. jeweils abgetrennt werden. Vorteil einer solchen vergleichsweise geringen Desinfektionsquote ist dabei beispielsweise, dass eine kürzere Einwirkzeit der UV-Strahlung und/oder eine niedrigere UV-Strahlungsintensität verwendet werden kann.

In einer bevorzugten Ausführungsform werden mindestens 99 % bezogen auf die Anzahl der im eingesaugten Volumenstrom befindlichen Keime desinfiziert, was 2-log-Stufen entspricht. Diese höhere Desinfektionsquote ist beispielsweise für den Schutz vor hoch-infektiösen viralen Krankheitserregern, bei denen eine geringere Virenanzahl zur Ansteckung bereits ausreicht, vorteilhaft. In einer ebenfalls bevorzugten Ausführungsform werden mindestens 99,9 % der im eingesaugten Volumenstrom befindlichen Keime desinfiziert, was 3-log-Stufen entspricht. Diese Ausführungsform ist beispielsweise dann vorteilhaft, wenn ein hoher Schutzbedarf zum Beispiel bei vulnerablen Personen(gruppen) erforderlich ist oder bei hoher, wechselnder Kontakthäufigkeit (zum Beispiel in Tresen in Praxen, in Kassenbereichen).

Zur Desinfektion der eingesaugten Luft umfasst die erfindungsgemäße Vorrichtung vorzugsweise mindestens eine UV- Strahlungsquelle.

Bevorzugt ist, dass diese mindestens eine UV-C-Strahlungsquelle mindestens anteilig Strahlung im UV-C-Bereich 280-100 nm emittiert. Je nach Art der Strahlungsquelle wird Strahlung über einen Wellenlängenbereich mit einem Intensitätsmaximum emittiert. Bevorzugt liegt das Intensitätsmaximum der mindestens einen UV-C-Strahlungsquelle im UV-C-Bereich zwischen 280 - 100 nm.

In einer bevorzugten Ausführungsform emittiert die mindestens eine UV-C-Strahlungsquelle mit einem Intensitätsmaximum im oberen UV-C-Bereich bei einer Wellenlänge zwischen 240 nm und 280 nm. In diesem Bereich wird erfahrungsgemäß eine besonders hohe Desinfektionswirkung erzielt.

In einer besonders bevorzugten Ausführungsform liegt das Intensitätsmaximum der emittierten Strahlung der mindestens einen UV-C-Strahlungsquelle im Bereich zwischen 250 und 270 nm. Bei 260 nm absorbiert die RNA besonders viel Strahlung (Absorptionsmaximum), wodurch eine besonders hohe Desinfektionswirkung zu erwarten ist.

Bevorzugt werden UV-Strahlungsquellen eingesetzt, die mindesten 50 %, bevorzugt mehr als 80 % der emittierten Strahlung in den oben aufgeführten Wellenlängenbereichen emittiert. Dies ist für die zielgerichtete Desinfektion besonders effizient. Die UV-Strahlungsquelle kann beispielsweise als Quecksilber-Entladungslampe oder LED-Leuchtmittel ausgebildet sein.

Die eine oder mehrere UV-Strahlungsquellen sind in der erfindungsgemäßen Vorrichtung vorzugsweise so angeordnet oder steuerbar, dass eine ausreichende Desinfektion der eingesaugten und vorbeiströmenden Luft erreicht wird. Um eine optimierte Bestrahlung über eine möglichst hohe Einwirkzeit an der UV-Strahlungsquelle zu gewährleisten, ist die UV-Strahlungsquelle langgestreckt, vorzugsweise im wesentlichen kreiszylindrisch mit homogener Abstrahlcharakteristik ausgebildet. Wird beispielsweise die eingesaugte und vorbeiströmende Luft in einem Zylinder geführt, in dem mindestens eine UV-Strahlungsquelle positioniert ist, kann die Strahlung über eine definierte Strecke auf die vorbeiströmende Luft einwirken. Dadurch kann die Strahlung ausreichend auf die mit der der Luft transportierten Keime einwirken und diese inaktivieren. Ist die oder sind die UV-Strahlungsquelle(n) entlang einer Symmetrieachse des Zylinders angeordneten und emittieren über 360° homogen über ihre gesamte Länge UV-Strahlung, kann eine besonders effektive Bestrahlung der zuströmenden Luft erreicht werden. Auch kann die in der Vorrichtung integrierte mindestens eine UV-Strahlungsquellen so ausgeführt sein, dass die Strahlungsintensität beispielsweise durch einen Regelkreis auf Basis einer sensorischen Ermittlung der erreichten Keiminaktivierung im Bereich der Luftauslasseinheit steuerbar ist, wodurch je nach Anwendungssituation die gewünschte Keiminaktivierung erreicht werden kann.

In einer bevorzugten Ausführungsform kann die UV-Desinfektion mit einer katalytischen Reinigungsstufe kombiniert sein. Dazu wird die Luftströmung vorzugsweise zumindest anteilig über einen Katalysator geleitet, wobei der Katalysator zumindest anteilig durch die UV-Strahlungsquelle bestrahlt werden kann. Dieser Katalysator kann dabei auf einem Wandabschnitt der Luftführung auf mindestens einem im Luftstrom integrierten Träger aufgebracht sein. Diese zusätzliche katalytische Reinigung kann beispielsweise Gerüche reduzieren oder Luftschadstoffe katalytisch deutlich besser als durch UV-desinfektion reduzieren.

In einer bevorzugten Ausführungsform wird die UV-desinfektion mit weiteren Desinfektions- und/oder Reinigungseinrichtungen kombiniert. So können in der erfindungsgemäßen Vorrichtung beispielsweise eine Ionisationseinrichtung integriert sein, die in einer Reaktionszone zwischen den Elektroden zu einem ionisierten Gas führt, was eine stark desinfizierende Wirkung hat. Auch kann die erfindungsgemäße Vorrichtung Luftfilter aufweisen. So kann zum Beispiel mindestens ein Grobfilter integriert sein, der die Luft zunächst vorreinigt, damit die Vorrichtungskomponenten vor Verunreinigungen geschützt werden. Auch können Filter, wie beispielsweise HEPA-Filter und Aktivkohlefilter integriert sein, womit sich beispielsweise geruchsbildende Partikel bzw. Gerüche sowie geringe Mengen an Ozon beseitigen oder Bakterien und Aerosole aus dem Luftstrom filtern lassen. Diese weiteren Desinfektions- und/oder Reinigungseinrichtungen können beispielsweise bezogen auf den Luftstrom räumlich hintereinander angeordnet sein.

Die so in die Vorrichtung eingesaugte und desinfizierte Luft wird vorzugsweise anschließend durch mindestens eine Luftaustrittsöffnung so ausgeblasen, dass sich eine Luftbarriere zwischen den am sich am Möbelstück befindenden Personen ausbildet. Dazu weist die Vorrichtung mindestens eine Austrittsöffnung für die eingesaugte und desinfizierte Luft auf. Die Positionierung der mindestens einen Austrittsöffnung kann dabei wie folgt sein:
Die mindestens eine Austrittsöffnung kann in der Mitte einer schlitzförmigen Lufteinlasseinheit, die ringförmig oder eckig ausgebildet ist, positioniert vorliegen. Das heißt in dieser Ausführungsform vorzugsweise, dass eine als Schlitz ausgebildete Eintrittsöffnung im weitesten Sinne einen Ring (zum Beispiel Kreis oder Oval) oder eine eckige Figur wie zum Beispiel Dreieck oder Polygon bildet. Dabei heißt im weitesten Sinne, dass die Ringform oder eckige Form nicht vollständig geschlossen sein muss, sondern auch Unterbrechungen wie Stabilisierungsstäbe aufweisen kann. Solche Unterbrechungen können beispielsweise aus konstruktiven Gründen zur Stabilisierung der Konstruktion vorteilhaft sein. Auch muss die Form nicht vollständig geschlossen, jedoch vorzugsweise als Ring oder Mehreck ausgestaltet sein. Die mindestens eine Austrittsöffnung ist in dieser Ausführungsform vorzugsweise mittig in Bezug auf die schlitzförmige Eintrittsöffnung positioniert. Mittig bzw. in der Mitte deckt dabei den gesamten Flächenbereich der von der schlitzförmigen Eintrittsöffnung umschlossenen Fläche ab.

Die mindestens eine Austrittsöffnung kann dabei kreisförmig, oval oder eckig (zum Beispiel dreieckig, viereckig) ausgebildet sein. Auch können statt einer Austrittsöffnung mehrere solcher Austrittsöffnungen, beispielsweise für mehrere Durchströmungskanäle durch die Vorrichtung oder zur Aufteilung bzw. Verteilung der Luftströmung mittig positioniert sein.

In weiteren bevorzugten Ausführungsformen der Erfindung liegt die mindestens eine Austrittsöffnung in der Mitte zwischen mehreren Eintrittsöffnungen positioniert vor. Dabei sind vorzugsweise mehrere separate kreisförmige, eckige oder schlitzförmige Eintrittsöffnungen um die mindestens eine Austrittsöffnung positioniert. In einer bevorzugten Ausführungsform sind dabei die Eintrittsöffnungen ringförmig beispielsweise als Löcher um die mindestens eine Austrittsöffnung positioniert.

In weiteren bevorzugten Ausführungsformen der Erfindung liegt die mindestens eine Austrittsöffnung zwischen zwei Eintrittsöffnungen positioniert vor. Dabei können die beiden separaten Eintrittsöffnungen wieder kreisförmig, eckig oder schlitzförmig ausgebildet sein. Die mindestens eine Austrittsöffnung ist vorzugsweise zwischen diesen beiden Eintrittsöffnungen auf einer gedachten Verbindungslinie positioniert.

In einer bevorzugten Ausführungsform der Erfindung sind die Eintritts- und/oder Austrittsöffnungen mit einem Schutz vor Einsaugen oder hineinfallen von Kontaminationen (zum Beispiel Materialien, Stoffen, Gegenständen oder Verunreinigungen) versehen. Durch das unbeabsichtigte Hineinfallen oder Einsaugen kann die Vorrichtung beschädigt werden oder verstopfen. Beispielsweise kann zum Schutz davor eine Siebabdeckung oder ein fließartiges Gewebe auf oder in den Öffnungen so integriert sein, dass die Maschenweite des Siebs die Luft mit möglichst geringem Druckverlust durchlässt, Kontaminationen einer bestimmten Größe jedoch nicht.

In einer weiteren bevorzugten Ausführungsform sind eine oder mehrere der Eintritts- und/oder Austrittsöffnungen einzeln oder gemeinsam verschließbar. Dadurch können diese Eintritts- und/oder Austrittsöffnungen beispielsweise verschlossen werden, wenn die Vorrichtung nicht in Betrieb ist oder wenn sich nicht überall am Tisch Personen befinden. Dadurch wird beispielsweise verhindert, dass in dieser Zeit Gegenstände oder Verunreinigungen in die Vorrichtung gelangen. In einer besonders bevorzugten Ausführungsform ist ein automatisierter Schließvorrichtung zum Verschließen der Eintritts- und/ oder Austrittsöffnung integriert. Automatisch bedeutet dabei, dass dieses Verschließen entweder programmierbar oder mechanisch (zum Beispiel Federdruck bei nachlassendem Luftstromdruck) ohne auslösende Interaktion durch einen Benutzer erfolgt.

Vorzugsweise wird durch die erfindungsgemäße Vorrichtung erreicht, dass mindestens 90 % (bezogen auf die Anzahl) der ausgestoßenen Aerosole einer Person nicht in die Raumzone einer anderen Person am Möbelstück auf direktem Weg transportiert werden. Dies wird insbesondere durch die gezielte Ausbildung der Luftbarriere (wie beispielsweise Dicke, Höhe und Form des Strömungsbands, Strömungsgeschwindigkeit) und der eingesaugten Luftmenge erreicht. Dazu wird die Luftbarriere bevorzugt so ausgebildet, dass die Luftbarriere bemessen auf der Gerade zwischen Kopfbereich der Personen am Möbelstück auf mindestens einer Dicke von 10 cm mindestens eine Strömungsgeschwindigkeit von 0,2 m/s, bevorzugt mindestens 0,5 m/s aufweist. Dadurch wird die Ausatemluft einer Person anteilig durch die Vorrichtung eingesaugt und anteilig durch die Luftbarriere von anderen Personen am Tisch weggelenkt. In einer bevorzugten Ausführungsform wird durch die Gestaltung und Steuerung der Vorrichtung verhindert, dass mehr als 99 % (bezogen auf die Anzahl) der ausgestoßenen Aerosole einer Person in die Raumzone einer anderen Person am Tisch auf direktem Weg transportiert werden. Dies kann beispielsweise dadurch erreicht werden, indem die eingesaugte Luftmenge erhöht wird und die dann ausgestoßene größere Luftmenge eine stärkere (im Sinne einer geringeren Durchdringbarkeit mit ausgestoßenen Aerosolen) Luftbarriere ausbildet. Dadurch wird eine besonders hohe Schutzwirkung für die Personen am Tisch erzielt.

In einer bevorzugten Ausführungsform befindet sich der Teil der erfindungsgemäßen Vorrichtung zur Desinfektion von Luft außer der Eintritts- und der Austrittsvorrichtung auf Höhe oder unterhalb der Möbeloberfläche. Durch diese Positionierung stört die Vorrichtung den Arbeits- und Sichtbereich nicht, anders als bei Geräten, die nur auf dem Tisch platzierbar sind. In einer besonders bevorzugten Ausführungsform ist dieser Teil der erfindungsgemäßen Vorrichtung zur Desinfektion von Luft zudem zumindest Teil der Tragkonstruktion für das Möbelstück und erhält dadurch eine zusätzliche Funktion. Dies ist besonders platzsparend.

In einer bevorzugten Ausgestaltung ist die erfindungsgemäße Vorrichtung zur Desinfektion von Luft kontaminationsgesteuert. Kontaminationsgesteuert bedeutet vorzugsweise, dass durch mindestens einen Sensor die Anzahl bzw. Menge an Keimen in der angesaugten, die Vorrichtung durchströmenden und/oder aus der Vorrichtung ausgestoßenen Luft ermittelt wird und nach dessen Detektionswert die UV-Reinigungsintensität des Gerätes automatisiert gesteuert wird.

Als Detektionsgröße werden vorzugsweise die Gesamtanzahl der Keime oder die Anzahl einer ausgewählten Gruppe oder einer bestimmten Art der Keime in der Luft bestimmt, die durch die erfindungsgemäße Vorrichtung zumindest anteilig abgetötet oder inaktiviert werden sollen. So können beispielsweise als Detektionsgröße die Anzahl der Coronaviren, Rhinoviren, Influenzaviren und/oder SARS-CoV-2-Viren genutzt werden.

Zur Bestimmung der Detektionsgröße (Kontamination) wird vorzugsweise ein Sensor verwendet, der geeignet ist, diese Art von Keimen in dem Konzentrationsbereich der Keime in der Ansaugluft zu detektieren. Geeignet sind hierzu beispielsweise sogenannte Biosensoren. Biosensoren sind beispielsweise Messfühler, die mit biologischen Komponenten ausgestattet sind. Diese werden in der biotechnologischen Messtechnik angewendet. Ein Biosensor besteht vorzugsweise aus einem biologischen Erkennungselement und einem physikalischen Sensor (Transducer), die sich in direktem Kontakt befinden. Nach Interaktion des zu testenden Stoffes mit der biologischen Komponente, entsteht zunächst ein biologisch-chemisches Signal, welches durch den Transducer in ein elektrisches oder optisches Signal umgewandelt wird.

In einer weiteren bevorzugten Ausgestaltung ist mindestens ein Sensor in der Vorrichtung so positioniert, dass er die Anzahl bzw. Menge an Keimen in der angesaugten Luft, das heißt auf der Wegstrecke von Lufteinlassöffnung bis Desinfektionseinheit, ermittelt, wobei dessen Detektionswert zur Steuerung der UV-Reinigungsintensität des Gerätes genutzt wird.

In einer alternativen Ausführungsform ist mindestens ein Sensor im Bereich des Luftauslasses integriert, der die Anzahl bzw. Menge an Keimen in der desinfizierten Luft, das heißt auf der Wegstrecke von Desinfektionseinheit bis zur Austrittsöffnung, bestimmt, wobei nach dessen Detektionswert die UV-Reinigungsintensität des Gerätes automatisiert gesteuert wird.

In einer besonders bevorzugten Ausführungsform ist mindestens ein Sensor im Bereich der Austrittsöffnung und mindestens ein Sensor im Bereich der Eintrittsöffnung integriert. Dadurch fließen sowohl die Kontaminationskonzentration der unbehandelten als auch der behandelten Luft ein, was eine Steuerung vereinfacht und durch die zusätzliche Steuergröße auch beispielsweise gegen (temporäre) Fehlmessung absichert.

Zur automatisierten Steuerung ist vorzugsweise mindestens eine Steuereinheit in der erfindungsgemäßen Vorrichtung integriert, die die detektierten Signale des oder der Sensoren auswertet und nach einem vorgegebenen Algorithmus die Reinigungsintensität der Vorrichtung steuert.

Unter UV-Reinigungsintensität ist vorzugsweise zu verstehen, wieviel Keime (Anzahl) durch die (automatisierte) Steuerung der erfindungsgemäßen Vorrichtung desinfiziert bzw. inaktiviert werden. Zur Einstellung der Reinigungsintensität des Gerätes können durch die mindestens eine Steuereinheit beispielsweise Ansaugvolumen und/oder die Intensität der UV-Strahlung zur Desinfektion der angesaugten Luft in der Vorrichtung gesteuert werden. Wird also eine geringe Kontamination in der angesaugten Luft ermittelt, wird beispielsweise die Menge an angesaugter Luft und/oder die UV-Strahlungsintensität reduziert. Wird hingegen ein hoher Wert ermittelt, wird beispielsweise die Ansaugluftmenge und/oder die UV-Strahlungsintensität erhöht.

In einer bevorzugten Ausführungsform ist dazu die Summe der emittierten UV-Intensität in der erfindungsgemäßen Vorrichtung steuerbar. Dazu kann beispielsweise die mindestens eine UV-Strahlungsquelle selbst in der Intensität der emittierten Strahlung, beispielsweise über die angelegte Spannung, steuerbar sein. Vorzugsweise kann auch ein Satz von mehreren Strahlungsquellen installiert sein, wobei die Anzahl der emittierenden Strahlungsquellen und darüber die Summe der insgesamt emittierten Strahlung einstellbar ist. Vorteil dieser Ausführungsform ist beispielsweise, dass bei geringer Kontamination weniger Energie für die UV-Strahlungsquelle(n) verbraucht wird.

Alternativ oder ergänzend kann die Reinigungsintensität über das Ansaugvolumen, also die Luftmenge, die durch die Vorrichtung geleitet wird, gesteuert werden. Dazu kann die Fördereinrichtung für die Ansaugluft (z.B. ein Ventilator) so reguliert werden, dass er mehr oder weniger Luft ansaugt und durch die Vorrichtung fördert.

Vorteil der Kontaminationssteuerung der Vorrichtung zur Desinfektion von Luft ist, dass eine Desinfektion jeweils im optimalen Arbeitsbereich liegt, heißt, genau die Desinfektionsintensität erfolgt, die zur Elimination der Keime erforderlich ist. Dies ist besonders effizient und damit energiesparend, da bei geringer oder keiner Keimbelastung die Reinigungsintensität und damit der Energieverbrauch des Gerätes gesenkt ist. Die Geräuschemission ist zudem mit der Reinigungsintensität kausal verbunden. Bei geringer Reinigungsintensität, also beispielsweise dann notwendigen geringeren Volumenströmen, sinkt typischerweise die Geräuschemission, was für eine angenehme Arbeitsatmosphäre in der Regel als förderlich empfunden wird.

In einer bevorzugten Ausführungsform ist die erfindungsgemäße Vorrichtung in oder an dem Gehäuse mit zumindest einem Bewegungssensor bzw. Näherungssensor ausgerüstet, der bevorzugt mit einer Steuereinheit verbunden ist oder mit einer Steuereinheit ausgerüstet ist, mit der mindestens die Luftfördereinheit, die Desinfektionseinheit und/oder die UV-Strahlungsquelle regulierbar und/oder einschaltbar und/oder ausschaltbar sind. So können beispielsweise die Luftfördereinheit und die Strahlungsquelle automatisch eingeschaltet werden, wenn sich eine oder mehrere Personen dem Tisch nähern und automatisch ausgeschaltet werden, wenn sich keine Personen am Tisch befinden. Dies hat beispielsweise den Vorteil, dass das Einschalten des Gerätes nicht vergessen wird. Zudem lässt sich auf diese Weise das Gerät besonders energiesparend betreiben, sodass die Komponenten, die eine Stromversorgung benötigen, nur dann in Betrieb gesetzt werden, wenn tatsächlich Personen sich am Tisch. Bevorzugt sind dazu mehrere Bewegungssensoren vorgesehen, mit denen (mehrere) Personen besonders zuverlässig erfasst werden.

In einem weiteren Aspekt betrifft die Erfindung ein System umfassend eine Vorrichtung zur Desinfektion von Luft und ein Möbelstück. Die Vorrichtung ist vorzugsweise so angeordnet, dass sie auf einer horizontalen Oberfläche des Möbelstücks ruht oder darin integriert ist, wobei das Möbelstück vorzugsweise so konfiguriert ist, dass es von mindestens zwei Personen benutzt werden kann, wobei es sich bei dem Möbelstück besonders bevorzugt um einen Tisch, einen Schreibtisch, einen Empfangstresen, eine Theke oder einen Tresen handelt.

Eine Vorrichtung, die auf einer horizontalen Oberfläche ruht, ist bevorzugt so beschaffen, dass zumindest ein Teil der Vorrichtung in Kontakt mit der horizontalen Oberfläche ist. Andere Teile der Vorrichtung können sich über oder unter der Oberfläche befinden, z.B. im Falle einer ganz oder teilweise in eine Tischplatte integrierten Vorrichtung. So kann es beispielsweise sein, dass die Blenden einer Eintrittsöffnung auf der horizontalen Fläche aufliegen, während andere Teile der Vorrichtung entweder unterhalb oder oberhalb der Fläche liegen.

In einer bevorzugten Ausführungsform der Erfindung geht eine Höhe der Luftauslasseinheit der Vorrichtung nicht mehr als 50 cm, vorzugsweise nicht mehr als 30 cm, noch bevorzugter nicht mehr als 15 cm, über eine Höhe der Oberfläche des Möbelstücks hinaus.

In einer bevorzugten Ausführungsform der Erfindung ist die Vorrichtung ein Teil des Möbelstücks. Insbesondere kann die Desinfektionseinheit als Säule ausgebildet sein, auf der eine Möbelstückoberfläche wie eine Tischplatte ruht.

In einer weiteren bevorzugten Ausführungsform der Erfindung hat die Vorrichtung die Form eines Möbelstücks. Beispielsweise können die Lufteinlasseinheit, Luftauslasseinheit und/oder Kanäle der Vorrichtung als Bohrungen in einem Möbelstück ausgebildet sein.

In einem weiteren Aspekt betrifft die Erfindung eine Verwendung der erfindungsgemäßen Vorrichtung oder des erfindungsgemäßen Systems zu einer Reduzierung eines Ansteckungsrisikos durch luftübertragene Viruserkrankungen zwischen mindestens zwei Personen, die sich sitzend oder stehend an einem Möbelstück befinden.

In einem weiteren Aspekt betrifft die Erfindung ein Verfahren für die Desinfektion von Luft mittels der erfindungsgemäßen Vorrichtung. Die Vorrichtung liegt auf eine horizontale Oberfläche eines Möbelstücks vor oder ist darin integriert. Das Verfahren umfasst die folgenden Schritte:
▪ zumindest teilweises Absaugen von Atemluft von einer ersten Person, die das Möbelstück benutzt, wobei die Atemluft durch eine Lufteinlasseinheit zu einer Desinfektionseinheit gefördert wird,
▪ Desinfektion der Atemluft in der Desinfektionseinheit, vorzugsweise mittels UV-C-Strahlung,
▪ Abgabe von desinfizierter Luft aus einer Luftauslasseinheit zur Bildung einer Luftbarriere, wobei die Luftbarriere so konfiguriert ist, dass sie die erste Person von einer zweiten Person trennt, die das Möbelstück ebenfalls benutzt.

In einer bevorzugten Ausführungsform der Erfindung ist eine Eintrittsöffnung der Lufteinlasseinheit so positioniert, dass sie weniger als 2 m, vorzugsweise weniger als 1,5 m, noch bevorzugter weniger als 0,7 m vom Kopf der ersten Person entfernt ist, wobei sich die Eintrittsöffnung vorzugsweise zwischen der ersten Person und der Luftauslasseinheit befindet.

Verschiedene Prozessparameter wie die Strömungsgeschwindigkeit der Luft durch die Vorrichtung, der Abstand der Vorrichtung zu einer Person, die Intensität der freigesetzten UV-Strahlung usw. können manuell oder automatisch eingestellt werden. Das Verfahren kann Schritte zur automatischen Einstellung der oben genannten Parameter unter Verwendung eines Timers, von Sensoren und/oder einer Steuereinheit umfassen, wie oben für die Vorrichtung beschrieben. Die oben für die Vorrichtung genannten bevorzugten Strömungsgeschwindigkeiten und Desinfektionsraten gelten selbstverständlich auch für das Verfahren.

Begriffe wie im Wesentlichen, ungefähr, etwa, ca., annähernd, nahezu usw. beschreiben vorzugsweise einen Toleranzbereich von weniger als ± 20 %, vorzugsweise weniger als ± 10 %, besonders bevorzugt weniger als ± 5 % und insbesondere weniger als ± 1 % und schließen den genauen Wert ein.

Der durchschnittliche Fachmann erkennt, dass technische Merkmale, Definitionen und Vorteile bevorzugter Ausführungsformen des erfindungsgemäßen Verfahrens auch für die erfindungsgemäßen Vorrichtung, Verwendung und System gelten, und umgekehrt.

### Kurzbeschreibung der Figuren

Fig. 1 zeigt eine typische Besprechungssituation zwischen zwei Personen.
Fig. 2 zeigt eine Besprechungssituation zwischen zwei Personen unter Verwendung einer Vorrichtung gemäß einer bevorzugten Ausführungsform der Erfindung.
Fig. 3 zeigt eine Besprechungssituation zwischen zwei Personen unter Verwendung einer Vorrichtung gemäß einer weiteren bevorzugten Ausführungsform der Erfindung.
Fig. 4 zeigt eine Besprechungssituation zwischen drei Personen unter Verwendung einer Vorrichtung gemäß einer weiteren bevorzugten Ausführungsform der Erfindung.
Fig. 5 zeigt schematisch eine beispielhafte Anordnung von Eintrittsöffnungen um eine Gruppe von Austrittsöffnungen.
Fig. 6 zeigt die Verwendung einer Vorrichtung mit einer Theke oder einem Tresen gemäß einer weiteren bevorzugten Ausführungsform der Erfindung.

### Detaillierte Beschreibung

Die Erfindung wird anhand der nachfolgenden Figuren näher beschrieben. Alle dargestellten Merkmale und deren Kombinationen sind nicht nur auf diese Ausführungsbeispiele und deren Ausgestaltungen begrenzt. Vielmehr sollen diese stellvertretend für weitere mögliche, aber nicht explizit als Ausführungsbeispiele dargestellte weitere Ausgestaltungen kombinierbar angesehen werden.

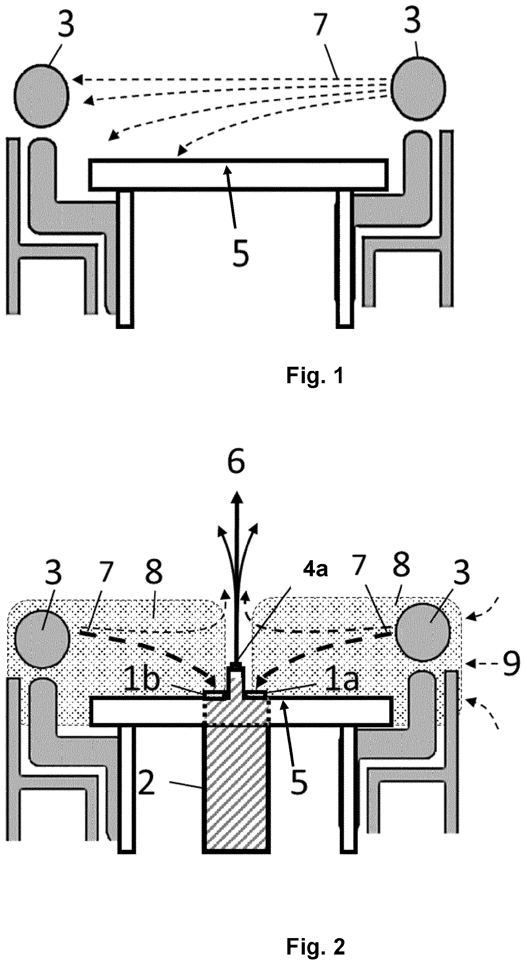

Figur 1 beschreibt eine typische Besprechungssituation, bei der zwei Personen 3 an einem Tisch mit einer Tischplatte 5 sitzen. Die Ausatem- und Sprechluft mit der darin enthaltenen Aerosolpartikel der einen Person strömt weitgehend ungehindert in Richtung der anderen Person (hier nur für eine Person vereinfacht dargestellt). Je nach Abstand zwischen den Personen erreicht dadurch eine erhebliche Anzahl von Aerosolen die jeweils andere Person beispielsweise im Mund-Nasenbereich, wodurch diese ausgestoßenen Aerosole der anderen Person zumindest anteilig mit eingeatmet werden oder über die Augenschleimhäute aufgenommen werden. Auch auftreffende Aerosole im Arbeitsbereich der Hand, zum Beispiel auf der Tischplatte, können so über die Hand in den Körper der Person eingetragen werden.

Figur 2 beschreibt die gleiche Gesprächssituation aus Figur 1, jedoch ist hier eine bevorzugte Ausführungsform der erfindungsgemäßen Vorrichtung 10 in Form eines Tischgeräts zur Desinfektion von Luft installiert, wobei durch die Gestaltung der Luftströmung durch diese Vorrichtung 10 eine Luftbarriere 6 zwischen den am Tisch sitzenden Personen 3 erzeugt wird. Durch diese erfindungsgemäße Luftbarriere 6 wird verhindert, dass vorzugsweise mehr als 90 % (bezogen auf die Anzahl) der Aerosole in der Ausatemluft 7 direkt zur jeweils anderen Person 3 gelangen. Direkt meint vorzugsweise in ungehinderter Strömungsrichtung wie in Figur 1 dargestellt, wenngleich die Luftströmung auch turbulent sein kann.

Die Vorrichtung 10 zur Desinfektion von Luft 10 ist bei dieser Ausführungsform in einem Tisch, insbesondere in einer Tischplatte, integriert. Dazu weist die Tischplatte 5 eine Aussparung auf. Die Desinfektionseinheit 2 befindet sich mindestens teilweise unterhalb der Tischplatte 5 und ragt mit der Vorrichtung 10 für die Eintrittsöffnungen 1a, 1b einer Lufteinlasseinheit 1 und Auslasseinheit 4 umfassend eine Austrittsöffnung 4a für die Luft durch die Tischplatte 5, so dass sich die Eintrittsöffnungen 1a, 1b und Austrittsöffnung oberhalb der Tischplatte befinden.

In der Desinfektionseinheit 2 ist oberhalb der Tischplatte 5 ein Bewegungssensor (nicht dargestellt) eingebaut, der über eine Steuereinheit die Desinfektionseinheit 2, insbesondere eine darin enthaltene UV-Strahlungsquelle, und eine Luftfördereinheit mit Platzierung der Personen 3 am Tisch eingeschaltet hat. Das heißt, der Bewegungssensor ist vorzugsweise so konfiguriert, dass er die Ankunft von Personen am Tisch erkennt und der Steuereinheit signalisiert, die entsprechenden Einheiten als Reaktion darauf einzuschalten.

Die Ausatemluft der beiden sich am Tisch befindenden Personen 3 wird mindestens anteilig in die erfindungsgemäße Vorrichtung 10 durch eine Luftfördereinheit ausgebildet als Ventilator (nicht dargestellt) eingesaugt. Dazu sind die Eintrittsöffnungen 1a, 1b so ausgerichtet, dass jeweils eine Eintrittsöffnung 1a bzw. 1b eine Gerade zwischen der Eintrittsöffnung und dem Mund- und Nasenbereich der am Tisch sitzenden Personen 3 bildet (Entfernung jeweils ca. 0,6 m). Konstruktiv sind die beiden Eintrittsöffnungen 1a, 1b identisch und werden in der Desinfektionseinheit 2 in einem Strömungskanal für die Luft zusammengeführt. Durch das Ansaugen der Luft durch die Eintrittsöffnungen 1a, 1b, hier jeweils mit identischen Ansaugvolumen durch die Eintrittsöffnungen 1a, 1b bildet sich eine dominierende Strömung vom Mund-Nasenbereich der jeweiligen Person 3 hin zur Eintrittsöffnungen 1a bzw. 1b aus, wodurch mindestens 10 Vol.-%, bevorzugt 30-70 Vol.-% der Ausatemluft jeweils beider Personen 3 in das Gerät mit der Ansaugluft eingesaugt wird.

Die in die Desinfektionseinheit 2 zusammengeführte Ansaugluft wird durch einen Durchströmungskanal zu der UV-Strahlungsquelle befördert und dort durch das Einwirken einer UV-C-Strahlung desinfiziert. Diese Strahlungsquelle wird in diesem Ausführungsbeispiel über eine automatisierte Steuerung auf Basis von einer durch zwei Sensoren ermittelten Keimanzahl in der Strahlungsintensität so geregelt, dass konstant mehr als 99 % bezogen auf die Anzahl der sich in der angesaugten Luft befindlichen Keime zerstört bzw. inaktiviert werden. Ein Sensor zur Ermittlung der Keimanzahl befindet sich dazu im zusammengeführten Strömungskanal zwischen der Lufteinlasseinheit und der Desinfektionseinheit 2 vor der UV-C-Strahlungseinwirkung, der andere Sensor auf einer hinter der Desinfektionseinheit unmittelbar vor der Austrittsöffnung der Luftauslasseinheit. Neben der UV-C-Intensität regelt die automatisierte Steuerung auch die Menge an angesaugtem Luftvolumen auf Basis der ermittelten Keimanzahl des Sensors auf der Ansaugseite vor der Strahlungseinwirkung der UV-C-Strahlungsquelle über die Regelung des Ventilators (hier die Luftfördereinheit). Dadurch wird je nach Keimanzahl (Kontaminationsgrad) erreicht, dass bei geringer Keimbelastung das Einsaugvolumen und damit die Geräuschentwicklung der Desinfektionseinheit 2 und der erzeugten Luftströmung sehr gering ist. Bei höherer Keimbelastung wird mehr Luftvolumen angesaugt und im Gerät desinfiziert, was dann entsprechend einen hohen Schutz für Ansteckung bietet.

Diese Luftbarriere 6 zwischen den beiden Personen 3 am Tisch wird durch das gerichtete Ausströmen der durch das Gerät geführten und desinfizierten Luft durch die Luftauslasseinheit 4 erreicht. Die Austrittsöffnung für die Luftauslasseinheit 4, die eingestellte Luftmenge und Strömungsgeschwindigkeit ist dabei so eingerichtet, dass sich eine Luftbarriere in Form eines Keils bzw. einer keilförmigen Wand ausbildet. Die Dicke dieser Wand nimmt mit zunehmender Entfernung von der Austrittsöffnung der Luftauslasseinheit 4 zu, die Strömungsgeschwindigkeit der Luftbarriere 6 hingegen ab. Die desinfizierte Luft kann durch die Ausrichtung und Form einer oder mehrerer geeigneter Austrittsöffnungen nach oben geleitet werden. Dem Fachmann sind geeignete Mittel bekannt, um einen Luftstrom nach oben zu leiten. Wie die dicken durchgehenden Pfeile zeigen, tritt mindestens eine Portion der desinfizierten Luft aus der Luftauslasseinheit 4 ungefähr senkrecht zur Tangentialebene der Tischoberfläche aus. Insbesondere bildet die Luftbarriere 6 einen Keil, die annähernd orthogonal zur Oberfläche des Tisches ist. Während alle Luftströme, die die Luftbarriere bilden, direkt nach oben gerichtet sein können, kann es auch bevorzugt sein, dass ein zentraler Teil der Luftauslasseinheit die desinfizierte Luft direkt nach oben richtet, während seitliche Teile die Luft in der gleichen orthogonalen Ebene zu den Seiten der Benutzer leiten. Dies wird im vorliegenden Beispiel dadurch erreicht, dass die Luft durch eine einzige schlitzförmige Austrittsöffnung geleitet wird. Eine solche Luftbarriere kann jedoch auch durch eine geeignete Anzahl von Düsen gebildet werden, die zum Beispiel in einer Reihe angeordnet sind und die unterschiedlich gerichteten Teile der Luftbarriere bilden.

In Richtung der Oberseite der Luftbarriere 6 beginnt der Luftstrom nach außen zu diffundieren. Die dicken gestrichelten Pfeile zeigen den Teil der ausgeatmeten Luft 7, der in die Lufteinlasseinheit 1 gesaugt wird. Dies wird insbesondere durch den Höhenunterschied zwischen der Luftauslasseinheit 4 und der Lufteinlasseinheit 1 begünstigt, der zusätzlich verhindert, dass die desinfizierte Luft direkt in die Vorrichtung 10 zurückkehrt und eine Endlosschleife bildet.

In der dargestellte Ausführungsform ragt die Austrittsöffnung für die Luftauslausseinheit 4 beispielsweise mit 20 cm höher als die Eintrittsöffnungen 1a, 1b der Lufteinlasseinheit 1 mit beispielsweise 3 cm über die Oberfläche der Tischplatte 5 heraus. Dadurch wird der Arbeits- und Sichtbereich der am Tisch sitzenden Personen 3 nicht signifikant eingeschränkt, jedoch das Vermischen von Ansaugluftströmung und Ausblasluftströmung deutlich vermindert.

Durch die ausgebildete Luftbarriere 6 wird verhindert, dass der Anteil an Ausatemluft 7, der nicht durch die Vorrichtung 10 eingesaugt wird, direkt in die Raumzone 8 der gegenübersitzenden Person strömt. Vielmehr wird dieser Anteil der Ausatemluft 7 durch die gerichtete Luftbarriere zum Beispiel nach oben abgelenkt. Die dünnere gestrichelte Linie der Fig. 2 zeigt den Teil der ausgeatmeten Luft 7, der nicht direkt in die Lufteinlasseinheit 1 gelangt. Durch die angepasste Strömungsgeschwindigkeit der Luftbarriere 6 entsteht ein ausreichender Unterdruck um die Luftbarriere, der diese verbleibende ausgeatmete Luft 7 nach oben, weg von den Benutzern, lenkt. Auf diese Weise wird eine unerwünschte Vermischung der ausgeatmeten Luft der beiden Benutzer sehr effektiv verhindert.

Durch die anteilige Ablenkung der Ausatemluft 7 können sich zwar die darin befindlichen Aerosole im angrenzenden Raum verteilen und letztlich auch durch die zuströmende Luft 9 in die Raumzone der anderen am Tisch sitzenden Person 3 gelangen. Die dafür benötigte Zeit und die zurückgelegte Wegstrecke ist jedoch um ein Vielfaches länger. Dabei sinkt die Infektiosität der sich eventuell mit den Aerosolen ausgestoßenen Anzahl an Viren mit längerer Zeit, da diese außerhalb des Wirts nur begrenzt lebensfähig sind. Zudem sinkt die Anzahl an schwebenden Aerosolen über die Zeit und Wegstrecke, so dass folglich das Ansteckungsrisiko deutlich verringert wird.

Durch die Kombination aus anteilig eingesaugter Atemluft und der Ablenkung der nicht eingesaugten Ausatemluft durch die ausgebildeter Luftbarriere 6 wird verhindert, dass mehr als 90 % der von einer Person 3 am Tisch ausgestoßenen Aerosole direkt in die Raumzone 8 der jeweils anderen Person 3 eindringen und dort von der Person 3 eingeatmet werden kann. Die Raumzone 8 ist schematisch in der Fig. 2 dargestellt und umfasst ein Volumen der Luft, welche von der jeweiligen Person direkt eingeatmet wird. Wie schematisch dargestellt, kann sich dieses Volumen in allen Richtungen rund um den Kopf und/oder Oberkörper der jeweiligen Person erstrecken. Das Volumen erstreckt sich insbesondere 0,5 Meter vor der Person. Eine etwas niedrigere seitliche und noch niedrigere rückwärtige und aufwärtige Erstreckung ist ebenfalls in dem Volumen inbegriffen. In diesem Beispiel erstreckt sich das Volumen etwa 0,3 m seitlich von jeder Person und etwa 0,15 m hinter ihr. Die nach oben gerichtete Erstreckung liegt hier ebenfalls bei 0,15 m. Wie in Fig. 2 zu sehen ist, nimmt die Raumzone jeder Person einen etwa gleich großen Teil der Luft auf der Oberfläche des Tisches ein.

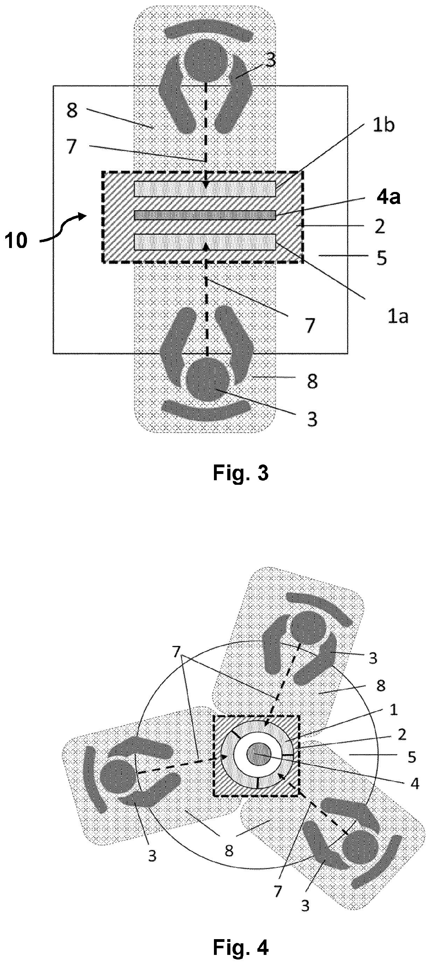

Figur 3 zeigt die gleiche Gesprächssituation und installierte Vorrichtung 10 zur Desinfektion von Luft wie Figur 2, hier jedoch in der Draufsicht von oben.

Durch die Eintrittsöffnungen 1a, 1b der Lufteinlasseinheit 1 wird die Ausatemluft 7 anteilig eingesaugt. Dazu ist die Form der Eintrittsöffnungen 1a und 1b sowie die Luftfördereinrichtung so eingerichtet, dass eine dominierende Strömung derart ausgebildet wird, dass die gewünschte Menge an Ausatemluft 7 in die Vorrichtung 10 eingesaugt wird.

In der beispielhaften Ausführungsform sind die Eintrittsöffnungen 1a und 1b in gleicher Weise viereckige ausgebildet. Die Austrittsöffnung 4a ist ebenfalls viereckig ausgebildet und zwischen den beiden Eintrittsöffnungen 1a, 1b der Lufteinlasseinheit 1 positioniert. Dabei ist die Austrittsöffnung 4a mit beispielsweise 1,5 cm schmaler ausgebildet als die beiden Eintrittsöffnungen 1a, 1b mit beispielsweise 2 cm. Auf diese Weise ist der Querschnitt der Austrittsöffnung 4a der Luftauslasseinheit 4 geringer als der Gesamtquerschnitt der Eintrittsöffnungen 1a, 1b der Lufteinlasseinheit 1. Auf diese Weise können jegliche zusammenlaufenden Kanäle, die die Luft zur Luftauslasseinheit 4 leiten, wie eine Düse wirken. Das Grundprinzip einer Düse ist, dass ein sich allmählich verengender Strömungsquerschnitt zu einer Erhöhung der Strömungsgeschwindigkeit führt. Dadurch hat die austretende desinfizierte Luft eine erhöhte Geschwindigkeit und eine besonders starke Tendenz, sich nach oben auszubreiten, wodurch sich die Dicke der Luftbarriere 6 auf der Kopfhöhe der Personen 3 erhöht. Die Luftbarriere 6 wird dadurch besonders effektiv.

Figur 4 zeigt eine andere Gesprächssituation im Vergleich zu Figur 1 bis 3. Hier sitzen 3 Personen an einem Besprechungstisch, der in runder Form ausgebildet ist.

Die Vorrichtung zur Desinfektion von Luft 10 befindet sich mittig in der Tischplatte integriert. Die Desinfektionseinheit 2 befindet sich unterhalb der Tischplatte 5. Die Lufteinlasseinheit 1 und Luftauslasseinheit 4 ragen durch eine Öffnung in der Tischplatte 5, so dass sich die Eintrittsöffnungen und Austrittsöffnungen oberhalb der Tischplatte befinden.

In dieser dargestellten Ausführungsvariante ist die rund ausgebildete Austrittsöffnung 4a in der Mitte der schlitzförmigen Eintrittsöffnung 1a, die ringförmig ist, positioniert. Das heißt Eintrittsöffnung 1a bildet einen geschlossenen Ring im Sinne eines Kreises um die Austrittsöffnung 4a. Dabei weist die Eintrittsöffnung 1a in dieser Ausführungsform 3 Stege zur Stabilisierung auf.

Durch die ringförmige Eintrittsöffnung 1a der Lufteinlasseinheit 1 wird die Ausatemluft 7 der am Tisch sitzenden Personen 3 anteilig eingesaugt. Die Eintrittsöffnung 1a sowie die Luftfördereinrichtung sind dazu erneut so eingerichtet, dass die Ausatemluft 7 durch eine dadurch erzeugte dominierende Strömung in gewünschte Menge, jedoch mindestens mit 10 Vol.-% in die Vorrichtung 10 eingesaugt wird.

In dieser Ausführungsform ragt die Austrittsöffnung 4a der Luftauslasseinheit 4 10 cm und die ringförmige Eintrittsöffnung 1 mit 1 cm über die Oberfläche der Tischplatte 5. Durch diese konstruktive Gestaltung können die Raumzonen 8 der am Tisch sitzenden Personen 3 und die Vermischung von angesaugtem und ausgestoßenem Luftvolumen besonders gut voneinander getrennt werden.

Durch die konstruktive Anordnung und Gestaltung von Eintrittsöffnung 1 und Austrittsöffnung 4 wird bei geeignet eingestellten Parametern (wie eingesaugtes und ausgestoßenes Luftvolumen) in dieser Ausführungsform eine Luftbarriere in Form eines auf dem Kopf stehenden, stumpfen Kegels gebildet. Dieser stumpfe Kegel sitzt dabei direkt auf der Austrittsöffnung 4a auf und verbreitet sich mit der nach oben strömenden, ausgestoßenen Luft. Mit zunehmender Höhe reduziert sich dabei die Strömungsgeschwindigkeit, wodurch im aufwärtigen, endenden Bereich der Luftbarriere ein gleitender Übergang in die Innenraumluft des gesamten Besprechungsraumes erfolgt.

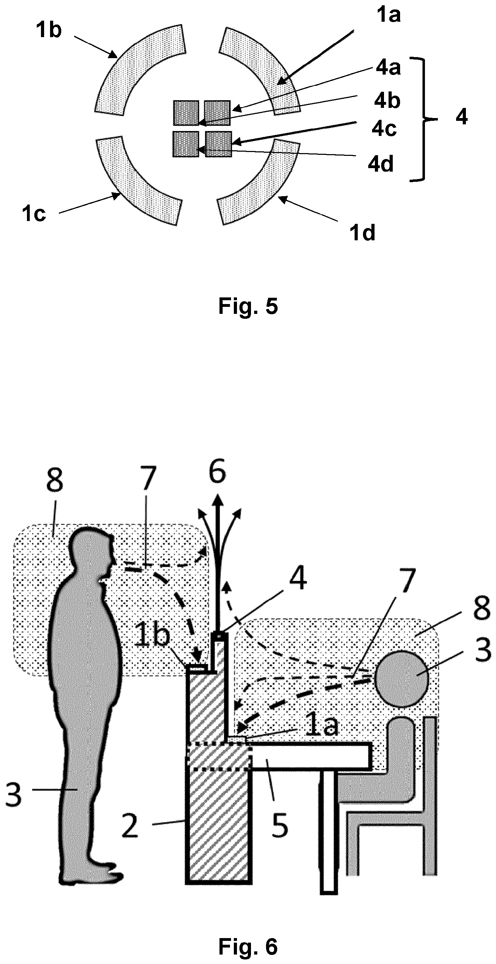

Figur 5 zeigt Ausgestaltungs- und Anordnungsform für die Lufteinlasseinheit 1 und Austrittsöffnungen 4a, 4b, 4c, 4d, welche beispielsweise für runde oder ovale Tische, insbesondere für eine vorgesehene Anzahl von bis zu vier Personen, geeignet ist.

Dabei ist eine viereckig angeordnete Luftauslasseinheit 4 unterteilt in vier viereckige Austrittsöffnungen 4a, 4b, 4c, 4d und in der Mitte einer schlitzförmigen, segmentierten Lufteinlasseinheit 1 positioniert.

Die Lufteinlasseinheit 1 bildet mit den vier Teilsegmenten 1a, 1b, 1c, 1d einen Ring um die Luftauslasseinheit 4. Die vier Teilsegmente 1a, 1b. 1c, 1d der Lufteinlasseinheit 1 stellen jeweils eine Eintrittsöffnung dar. Das jeweils durch die Teilsegmente bzw. Eintrittsöffnungen der Lufteinlasseinheit 1 angesaugte Luftvolumen wird in die Desinfektionseinheit 2 zusammengeführt und durch einen Durchströmungskanal zu der UV-Strahlungsquelle befördert und dort durch das Einwirken einer UV-C-Strahlung desinfiziert. Nach der Desinfektion vor Erreichen der Luftauslasseinheit 4 wird der Luftstrom auf die vier Teilsegmente der Luftauslasseinheit 4a, 4b, 4c, 4d in diesem Ausführungsbeispiel gleichmäßig aufgeteilt.

Figur 6 beschreibt beispielhaft eine Ausführungsvariante an einem als Empfangstisch bzw. als Tresen gebildete Möbelstück, wobei eine Person 3 an diesem Möbelstück sitzt und eine Person 3 vor diesem Tresen steht. Im Sinne der Erfindung wird das Möbelstück von der sitzenden und stehenden Person verwendet. In dem Tresen ist eine Vorrichtung 10 zur Desinfektion von Luft gemäß einer bevorzugten Ausführungsform der Erfindung installiert, wobei durch die erfindungsgemäße Gestaltung der Luftströmung durch diese Vorrichtung eine Luftbarriere 6 zwischen der am Tresen sitzenden und der stehenden Personen 3 erzeugt wird.

Die Vorrichtung zur Desinfektion von Luft 10 bildet die über die Arbeitsplatte der sitzenden Person, hier als Tresenplatte 5 bezeichnet, hinausragende Seiten- bzw. Vorderwand des Tresens, vor der sich die stehende Person befindet. Die Tresenplatte 5 kann dabei an die Vorrichtung zur Desinfektion von Luft 10 montiert sein. Alternativ kann die Tresenplatte 5 analog der Ausführungsvarianten der Figur 2 bis 4 eine Aussparung aufweisen, sich die Desinfektionseinheit 2 unterhalb der Tresenplatte 5 beispielsweise als Stütze für die Tresenplatte 5 befinden und die Lufteinlasseinheit 1 und Luftauslasseinheit 4 für die Luft durch die Tresenplatte 5 geführt sein, so dass sich die Lufteinlasseinheit 1 und Luftauslasseinheit 4 oberhalb der Tresenplatte befinden.

In dieser Ausführungsvariante sind oberhalb der Tresenplatte 5 zwei Bewegungssensoren (nicht dargestellt) in der Weise eingebaut, dass zum einen über eine Steuereinheit die Luftfördereinheit und die UV-Strahlungsquelle der Vorrichtung 10 eingeschaltet wird, sobald sich mindestens eine Person an den Tresen setzt oder vor den Tresen stellt. Zum anderen wird über eine Steuereinheit einen Lufteinlass aus der Eintrittsöffnung 1a erst dann freigegeben, wenn eine Person am Tresen sitzt, die Eintrittsöffnung 1b erst freigegeben sobald sich eine Person 3 vor dem Tresen befindet. Dazu kann die Steuereinheit einen gesteuerten Verschluss oder Abdichtklappe im Luftkanal hinter jeder Eintrittsöffnung 1a und 1b steuern. Dadurch wird erreicht, dass die Luftfördereinheit und die UV-Strahlungsquelle der Desinfektionseinheit 2 der Vorrichtung 10 nur dann arbeitet und dass nur dann und nur von der Seite Luft eingesaugt wird, an der sich eine Person befindet. Dieser automatisierte Arbeitsbetrieb ist besonders effizient, energiesparend und beugt Bedienfehler wie beispielsweise das Vergessen des Einschaltens vor.

Die Ausatemluft der beiden sich am Tresen befindenden Personen 3 wird anteilig in die Vorrichtung 10 durch eine Luftfördereinheit eingesaugt. Konstruktiv sind die beiden Eintrittsöffnungen 1a, 1b so ausgerichtet, dass bei der typischen dargestellten Gesprächssituation jeweils eine Gerade zwischen der Eintrittsöffnung und dem Mund- und Nasenbereich von ca. 0,7 m resultiert. Dazu ist die Eintrittsöffnung 1b um 0,1 - 0,6 m höher als die Eintrittsöffnung 1a positioniert, um den durchschnittlichen Höhenunterschied einer sitzenden und stehenden Person zu kompensieren. In der dargestellte Ausführungsform ragt die Austrittsöffnung 4a der Luftauslasseinheit mit 50 cm, die Eintrittsöffnung 1a für die sitzende Person 1 cm und die Eintrittsöffnung für die stehende Person 3 um 40 cm über die Oberfläche der Tresenplatte hinaus. Durch das Ansaugen der Luft durch die Eintrittsöffnungen 1a, 1b, bildet sich jeweils eine dominierende Strömung vom Mund-Nasenbereich der Person 3 hin zur jeweiligen Eintrittsöffnung 1a bzw. 1b aus, wodurch in dieser Ausführungsvariante bevorzugt 30-70 Vol.-% der Ausatemluft jeweils beider Personen 3 in die Vorrichtung 10 eingesaugt wird

Die angesaugte Luft durch die Eintrittsöffnungen 1a, 1b wird in der Vorrichtung 10 hinter dem steuerbaren Verschluss zusammengeführt und durch einen gemeinsamen Durchströmungskanal zu der UV-Strahlungsquelle befördert und dort durch das Einwirken einer UV-C-Strahlung desinfiziert. Analog der Beschreibung zu Figur 2 kann die UV-Strahlungsquelle über eine automatisierte Steuerung auf Basis von durch zwei Sensoren ermittelten Keimanzahl so geregelt werden, dass konstant mehr als 99 % bezogen auf die Anzahl der sich in der angesaugten Luft befindlichen Keime zerstört bzw. inaktiviert werden.

Die erfindungsgemäße Luftbarriere 6 zwischen den beiden Personen 3 am Tresen wird durch das gerichtete Ausströmen der durch die Desinfektionseinheit 2 geführten und desinfizierten Luft durch die Luftauslasseinheit 4 erreicht. Die Form der Austrittsöffnung 4a, die eingestellten Luftmenge und Strömungsgeschwindigkeit ist dabei so eingerichtet, dass sich eine Luftbarriere in Form einer Wand ausbildet, deren Dicke mit zunehmender Entfernung von der Austrittsöffnung 4a zu- und die Strömungsgeschwindigkeit abnimmt. Durch die ausgebildete Luftbarriere 6 wird verhindert, dass der Anteil an Ausatemluft 7, der nicht durch die Vorrichtung 10 eingesaugt wird, direkt in die Raumzone 8 der sich gegenüber befindender Person strömt. Vielmehr wird dieser Anteil der Ausatemluft 7 durch die gerichtete Luftbarriere zum Beispiel nach oben abgelenkt. In diesem Ausführungsbeispiel wird durch die Steuerung der Luftbarriere 6 verhindert, dass mehr als 95 % (bezogen auf die Anzahl) der Aerosole in der Ausatemluft 7 direkt zur jeweils anderen Person 3 gelangen.

**Bezugszeichenliste:**

| | |
|---|---|
| **1** | Lufteinlasseinheit |
| **1a, 1b, 1c, 1d** | Eintrittsöffnung |
| **2** | Desinfektionseinheit |
| **3** | Person am Tisch, Tresen oder Theke |
| **4** | Luftauslasseinheit |
| **4a, 4b, 4c, 4d** | Austrittsöffnung |
| **5** | Oberfläche des Möbelstücks, insbesondere Tisch-, Tresen- oder Thekenplatte |
| **6** | Luftbarriere |
| **7** | Ausatemluft |
| **8** | Ein-/ Ausatembereich (Raumzone) einer Person |
| **9** | Zuströmende Luft in Ein-/Ausatembereich |
| **10** | Vorrichtung |

## Patentansprüche

1. Vorrichtung (10) zur Desinfektion von Luft umfassend mindestens eine Lufteinlasseinheit (1), eine Luftfördereinheit, eine Desinfektionseinheit (2) und eine Luftauslasseinheit (4),
**dadurch gekennzeichnet, dass**
die Lufteinlasseinheit (1) und die Luftfördereinheit für das Ansaugen von Ausatemluft (7) von mindestens zwei Personen (3) konfiguriert sind,
die Luftfördereinheit weiterhin für die Förderung der Ausatemluft (7) von der Lufteinlasseinheit (1) über die Desinfektionseinheit (2) zu der Luftauslasseinheit (4) konfiguriert ist und
die Luftauslasseinheit (4) für die Bildung einer Luftbarriere (6) konfiguriert ist,
wobei die Lufteinlasseinheit (1) in Umfangsrichtung um die Luftauslasseinheit (4) angeordnet vorliegt, sodass die Luftauslasseinheit mindestens teilweise von der Lufteinlasseinheit umgeben ist.

2. Vorrichtung gemäß Anspruch 1
**dadurch gekennzeichnet, dass**
die Luftbarriere (6) eine vertikale konische Form mit einer nach unten gerichteten Spitze, eine vertikale Keilform oder eine Kombination davon aufweist.

3. Vorrichtung (2) gemäß einem der vorherigen Ansprüche
**dadurch gekennzeichnet, dass**
die Luftauslasseinheit (4) für das Ausblasen von desinfizierter Luft nach oben konfiguriert ist, insbesondere wenn die Vorrichtung (10) auf einer Oberfläche eines Möbelstücks (5) positioniert vorliegt oder darin integriert ist.

4. Vorrichtung (2) gemäß einem der vorherigen Ansprüche
**dadurch gekennzeichnet, dass**
die Lufteinlasseinheit (1) mehrere Eintrittsöffnungen (1a, 1b) umfasst, wobei vorzugsweise eine Eintrittsöffnung pro Person vorgesehen ist.

5. Vorrichtung (10) nach einem der vorherigen Ansprüche,
**dadurch gekennzeichnet, dass**
die Luftfördereinheit und die Luftauslasseinheit (4) für eine Strömungsgeschwindigkeit aus der Luftauslasseinheit (4) im Bereich von 0,2 m/s bis 2,5 m/s ausgelegt sind.

6. Vorrichtung (10) nach einem der vorherigen,
**dadurch gekennzeichnet, dass**
die Vorrichtung (10) eine Steuereinheit umfasst, wobei die Steuereinheit dafür konfiguriert ist, die Vorrichtung (10) nach einer Kontaminationsintensität mindestens teilweise automatisiert zu steuern, wobei
vorzugsweise eine Keimanzahl in eingesaugter Luft auf einer Wegstrecke zwischen der Lufteinlasseinheit (1) und der Desinfektionseinheit (2) und/oder in desinfizierter Luft auf einer Wegstrecke zwischen der Desinfektionseinheit (2) und der Luftauslasseinheit (4) durch mindestens einen Sensor ermittelt wird und
die Steuereinheit für die Auswertung von Signalen von dem mindestens einen Sensor und für die Steuerung der Luftfördereinheit und/oder der Desinfektionseinheit (2) nach einem vorgegebenen Algorithmus konfiguriert ist, wobei der Algorithmus für eine Inaktivierung eines vorgegebenen Anteils oder Anzahl von Keimen in der eingesaugten Luft ausgelegt ist.

7. Vorrichtung (10) nach einem der vorherigen Ansprüche,
**dadurch gekennzeichnet, dass**
die Vorrichtung (10) für die Verwendung auf oder integriert in einer Oberfläche eines Möbelstücks (5) konfiguriert ist,
wobei vorzugsweise die Luftauslasseinheit (4) in dergleichen oder einer höheren horizontalen Ebene als die Lufteinlasseinheit (1) vorliegt, insbesondere wenn die Vorrichtung (10) auf die Oberfläche des Möbelstücks (5) positioniert oder darin integriert ist.

8. Vorrichtung (10) gemäß dem vorherigen Anspruch
**dadurch gekennzeichnet, dass**
die Lufteinlasseinheit (1) und die Luftauslasseinheit (4) nicht mehr als 50 cm, vorzugsweise nicht mehr als 30 cm, noch bevorzugter nicht mehr als 15 cm höher als die Oberfläche des Möbelstücks (5) sind, insbesondere wenn die Vorrichtung (10) auf die Oberfläche des Möbelstücks (5) positioniert oder darin integriert ist.

9. Vorrichtung (10) gemäß einem der vorherigen Ansprüche
**dadurch gekennzeichnet, dass**
die Desinfektionseinheit (2) eine UV-Strahlungsquelle umfasst, wobei die UV-Strahlungsquelle für das mindestens anteilige Emittieren von Strahlung mit einer Wellenlänge zwischen 100 - 280 nm konfiguriert ist.

10. Vorrichtung (10) nach einem der vorherigen Ansprüche,
**dadurch gekennzeichnet, dass**
die Vorrichtung (10) für das Ansaugen mindestens 10 Vol.-% der gesamten Ausatemluft (7) von mindestens zwei Personen (3) konfiguriert ist, insbesondere wenn die mindestens zwei Personen (3) um ein Möbelstück (5) sitzen oder stehen, auf dessen Oberfläche die Vorrichtung (10) aufgestellt oder in dessen Oberfläche die Vorrichtung (2) integriert ist.

11. Vorrichtung (10) nach einem der vorherigen Ansprüche,
**dadurch gekennzeichnet, dass**
die Vorrichtung für die Desinfizierung mindestens 90 % von Keimen bezogen auf die Anzahl, die sich in einem eingesaugten Volumenstrom befinden konfiguriert ist.

12. Vorrichtung (10) nach einem der vorherigen Ansprüche,
**dadurch gekennzeichnet, dass**
durch die Luftbarriere (6) mindestens 90 % einer Anzahl von ausgestoßenen Aerosolen einer ersten Person von ihrem Weg zu einer zweiten Person umgelenkt werden.

13. System umfassend eine Vorrichtung (10) gemäß einem der vorherigen Ansprüche und ein Möbelstück (5),
**dadurch gekennzeichnet, dass**
die Vorrichtung (10) vorzugsweise so angeordnet ist, dass sie auf einer horizontalen Oberfläche des Möbels (5) ruht oder darin integriert ist, wobei das Möbelstück vorzugsweise so konfiguriert ist, dass es von mindestens zwei Personen (3) benutzt werden kann, wobei es sich bei dem Möbelstück besonders bevorzugt um einen Tisch, einen Schreibtisch, einen Empfangstresen, eine Theke oder einen Tresen handelt,
wobei vorzugsweise
eine Höhe der Lufteinlasseinheit (1) und eine Höhe der Luftauslasseinheit (4) der Vorrichtung (10) nicht mehr als 50 cm, vorzugsweise nicht mehr als 30 cm, noch bevorzugter nicht mehr als 15 cm, über eine Höhe der Oberfläche des Möbelstücks (5) hinausgehen.

14. Verwendung der Vorrichtung (10) nach einem oder mehreren der Ansprüche 1 bis 10 oder des Systems nach einem der Ansprüche 11 - 12 zu einer Reduzierung eines Ansteckungsrisikos durch luftübertragene Viruserkrankungen zwischen mindestens zwei Personen (3), die sich sitzend oder stehend an einem Möbelstück (5) befinden.

15. Verfahren für die Desinfektion von Luft mittels einer Vorrichtung gemäß einem der Ansprüche 1 - 12
**dadurch gekennzeichnet, dass** die Vorrichtung (10) auf eine horizontale Oberfläche eines Möbelstücks vorliegt oder darin integriert ist und das Verfahren die folgenden Schritte umfasst:
- zumindest teilweises Absaugen von Atemluft (7) von einer ersten Person, die das Möbelstück benutzt, wobei die Atemluft (7) durch eine Lufteinlasseinheit (1) zu einer Desinfektionseinheit (2) gefördert wird,
- Desinfektion der Atemluft (7) in der Desinfektionseinheit (2), vorzugsweise mittels UV-C-Strahlung,
- Abgabe von desinfizierter Luft aus einer Luftauslasseinheit (4) zur Bildung einer Luftbarriere (6), wobei die Luftbarriere so konfiguriert ist, dass sie die erste Person von einer zweiten Person trennt, die das Möbelstück ebenfalls benutzt.
